(19) Europäisches Patentamt
European Patent Office
Office européen des brevets

(11) Publication number: 0 186 118
B1

(12) EUROPEAN PATENT SPECIFICATION

(45) Date of publication of patent specification: 09.05.90

(21) Application number: 85116162.0

(22) Date of filing: 18.12.85

(51) Int. Cl.[5]: C 07 C 205/45, C 07 C 201/06, C 07 C 255/50, A 01 N 35/06, A 01 N 41/06, A 01 N 41/10, C 07 C 205/49, C 07 C 317/24, C 07 C 317/44

# (54) Certain 2-(2'nitrobenzoyl)-1,3-cyclohexanediones.

(30) Priority: 20.12.84 US 683900

(43) Date of publication of application:
02.07.86 Bulletin 86/27

(45) Publication of the grant of the patent:
09.05.90 Bulletin 90/19

(84) Designated Contracting States:
AT BE CH DE FR GB IT LI NL SE

(56) References cited:
EP-A-0 007 243
EP-A-0 017 195
EP-A-0 135 191
EP-A-0 137 963
EP-A-0 162 336
EP-A-0 186 117

**The file contains technical information submitted after the application was filed and not included in this specification**

(73) Proprietor: STAUFFER CHEMICAL COMPANY
Westport Connecticut 06881 (US)

(72) Inventor: Carter, Charles Garvie
1240 Willard Street
San Francisco Calif. 94117 (US)

(74) Representative: Kraus, Walter, Dr. et al
Patentanwälte Kraus, Weisert & Partner
Thomas-Wimmer-Ring 15
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 186 118 B1

## Description

Background of the Invention

Compounds having the structural formula

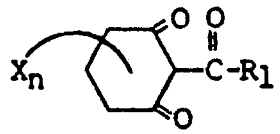

wherein X can be an alkyl, n can be 0, 1, or 2, and $R_1$ can be phenyl or substituted phenyl are described in Japanese Patent Application 84632—1974 as being intermediates for the preparation of herbicidal compounds of the formula

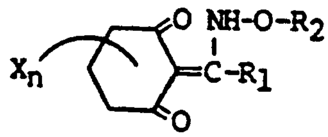

wherein $R_1$, X, and n are as defined above, and $R_2$ is alkyl, alkenyl, or alkynyl. Specifically taught herbicidal compounds of this latter group are those where n is 2, X is 5,5-dimethyl, $R_2$ is allyl and $R_1$ is phenyl, 4-chlorophenyl or 4-methoxyphenyl.

The precursor intermediates for these three specifically taught compounds have no or almost no herbicidal activity.

European Patent Application No. 83 102 599.4 was published October 5, 1983 and relates to certain novel 2-(2-substituted benzoyl)-cyclohexane-1,3-diones as herbicides. The compounds have the following structural formula

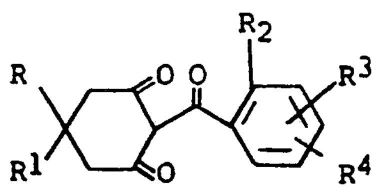

wherein R and $R^1$ are hydrogen or $C_1$—$C_4$ alkyl; $R^2$ is chlorine, bromine, or iodine; $R^3$ is hydrogen or halogen; and $R^4$ is hydrogen, chlorine, bromine, iodine, $C_1$—$C_4$ alkyl, $C_1$—$C_4$ alkoxy, nitro or trifluoromethyl.

Description of the Invention

This invention relates to 2-(2-nitrobenzoyl)-1,3-cyclohexanediones and their use as herbicides.

The compounds have a nitro substitution in the 2-position of the phenyl moiety of their compounds. The nitro substitution imparts exceptional herbicidal activity to the compounds of this invention.

One embodiment of this invention is an herbicidal composition comprising an herbicidally active 2-(2-nitrobenzoyl)-1,3-cyclohexanedione and an inert carrier therefor. The 4-, 5- and 6-positions of the 1,3-cyclohexanedione moiety can be substituted, preferably with the groups hereinafter recited. More preferably, the 1,3-cyclohexanedione moiety has no substitution or the 4- or 6-positions are substituted with one or two methyl groups. The 3-, 4- and 5-positions of the benzoyl moiety can be substituted, preferably with the groups hereinafter recited.

Also embodied within the scope of this invention are novel compounds having the following structural formula

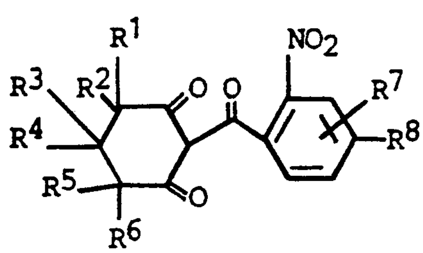

wherein

$R^1$ is hydrogen or $C_1$—$C_4$ alkyl, preferably $C_1$—$C_2$ alkyl, more preferably methyl, most preferably $R^1$ is

hydrogen or methyl;

$R^2$ is hydrogen; $C_1$—$C_4$ alkyl, preferably $C_1$—$C_2$ alkyl, more preferably methyl or

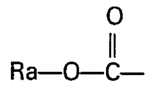

wherein

$R^a$ is $C_1$—$C_4$ alkyl, most preferably $R^2$ is hydrogen or methyl; or

$R^1$ and $R^2$ together are alkylene having 3 to 6 carbon atoms;

$R^3$ is hydrogen or $C_1$—$C_4$ alkyl, preferably $C_1$—$C_2$ alkyl, more preferably methyl; most preferably $R^3$ is hydrogen or methyl;

$R^4$ is hydrogen or $C_1$—$C_4$ alkyl, preferably $C_1$—$C_2$ alkyl, more preferably methyl; most preferably $R^4$ is hydrogen or methyl;

$R^5$ is hydrogen or $C_1$—$C_4$ alkyl, preferably $C_1$—$C_2$ alkyl, more preferably methyl; most preferably $R^5$ is hydrogen or methyl;

$R^6$ is hydrogen or $C_1$—$C_4$ alkyl, preferably $C_1$—$C_2$ alkyl, more preferably methyl, most preferably $R^6$ is hydrogen;

$R^7$ and $R^8$ independently are (1) hydrogen; (2) halogen, preferably chlorine, fluorine or bromine; (3) $C_1$—$C_4$ alkyl, preferably methyl; (4) $C_1$—$C_4$ alkoxy, preferably methoxy; (5) $OCF_3$; (6) cyano; (7) nitro; (8) $C_1$—$C_4$ haloalkyl, more preferably trifluoromethyl; (9) $R^bSO_n$— wherein n is the integer 0, 1 or 2, preferably 2; and

$R^b$ is

(a) $C_1$—$C_4$ alkyl, preferably methyl;

(b) $C_1$—$C_4$ alkyl substituted with halogen or cyano, preferably chloromethyl, trifluoromethyl or cyanomethyl;

(c) phenyl; or

(d) benzyl;

(10) —$NR^cR^d$ wherein $R^c$ and $R^d$ independently are hydrogen or $C_1$—$C_4$ alkyl; (11) $R^eC(O)$— wherein $R^e$ is $C_1$—$C_4$ alkyl or $C_1$—$C_4$ alkoxy; or (12) —$SO_2NR^cR^d$ wherein $R^c$ and $R^d$ are as defined, with the proviso that $R^7$ is not attached to the 6-position.

Preferably, $R^7$ is in the 3-position. Most preferably $R^7$ is hydrogen or $C_1$—$C_4$ alkoxy and $R^8$ is hydrogen, chlorine, bromine, fluorine, $CF_3$, or $R^bSO_2$ wherein $R^b$ is $C_1$—$C_4$ alkyl, preferably methyl.

The term "$C_1$—$C_4$ alkyl" includes methyl, ethyl, n-propyl, isopropyl, n-butyl, sec-butyl, isobutyl and t-butyl. The term "halogen" includes chlorine, bromine, iodine and fluorine. The terms "$C_1$—$C_4$ alkoxy" includes methoxy, ethoxy, n-propoxy, isopropoxy, n-butoxy, sec-butoxy, isobutoxy and t-butoxy. The term "haloalkyl" includes the eight alkyl groups with one or more hydrogens replaced by chlorine, bromine, iodine or fluorine.

Salts of the above-described compounds (as defined hereinafter) are also the subject of the instant invention.

The compounds of this invention can have the following four structural formulae because of tautomerism:

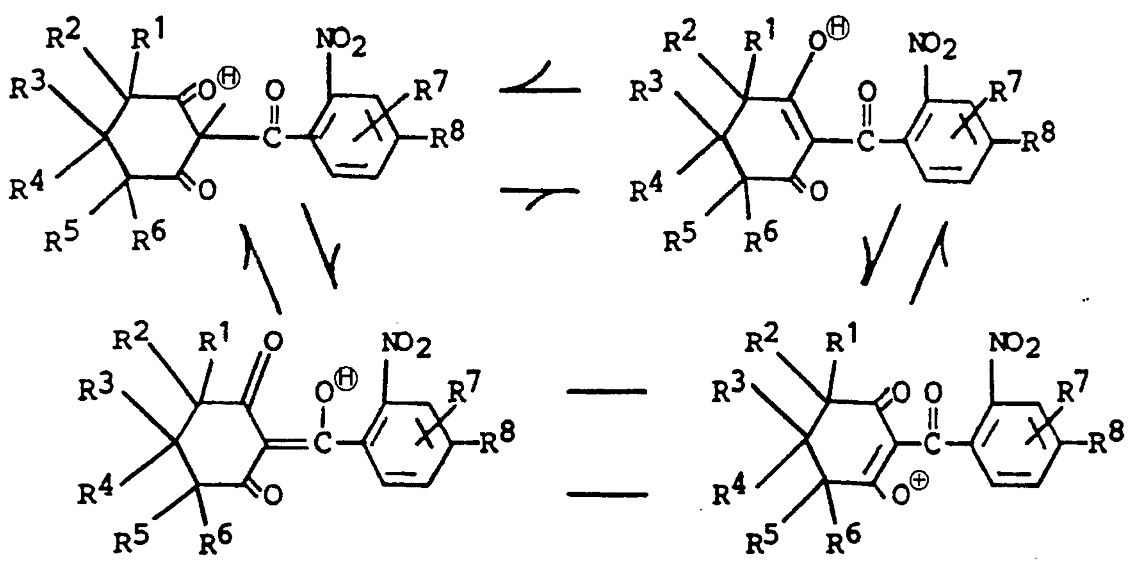

wherein $R^1$, $R^2$, $R^3$, $R^4$, $R^5$, $R^6$, $R^7$ and $R^8$ are as defined above.

The circled proton on each of the four tautomers is reasonably labile. These protons are acidic and can be removed by any base to give a salt having an anion of the following four resonance forms:

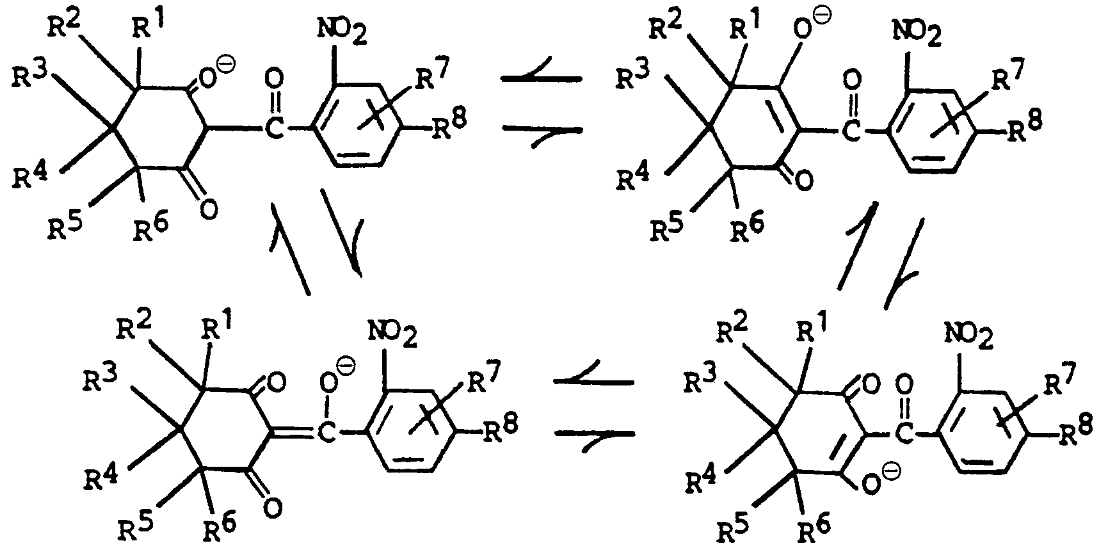

wherein $R^1$, $R^2$, $R^3$, $R^4$, $R^5$, $R^6$, $R^7$ and $R^8$ are as defined above.

Examples of cations of these bases are inorganic cations such as alkali metals e.g. lithium, sodium, and potassium organic cations such as substituted ammonium, sulfonium or phosphonium wherein the substituent is an aliphatic or aromatic group.

The compounds of this invention and their salts are active herbicides of a general type. That is, they are herbicidally effective against a wide range of plant species. The method of controlling undesirable vegetation of the present invention comprises applying an herbicidally effective amount of the above-described compounds to the area where control is desired.

The compounds of the present invention can be prepared by the following two-step general method.

The process proceeds via the production of an enol ester intermediate as shown in reaction (1). The final product is obtained by rearrangement of the enol ester as shown in reaction (2). The two reactions may be conducted as separate steps by isolation and recovery of the enol ester using conventional techniques prior to conducting step (2), or by addition of a cyanide source to the reaction medium after the formation of the enol ester, or in one step by inclusion of the cyanide source at the start of reaction (1).

1) 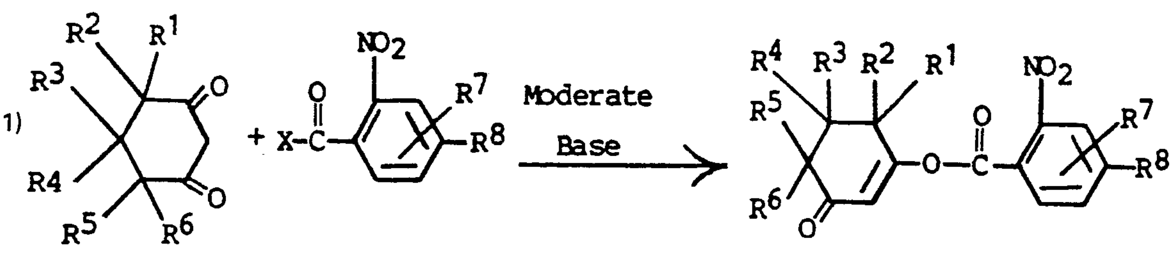


wherein $R^1$ through $R^8$ and moderate base are as defined and X is halogen, preferably chlorine, $C_1$—$C_4$ alkyl-C(O)—O—, $C_1$—$C_4$ alkoxy-C(O)—O— or

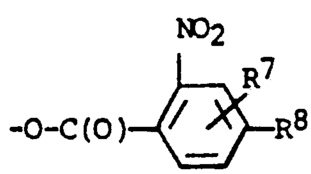

wherein $R^7$ and $R^8$ in this portion of the molecule are identical with those in the reactant shown above and the moderate base is as defined, preferably tri-$C_1$—$C_6$ alkylamine, pyridine, alkali metal carbonate or alkali metal phosphate.

Generally, in step (1) mole amounts of the dione and substituted benzoyl reactant are used, along with a mole amount or excess of the base. The two reactants are combined in an organic solvent such as methylene chloride, toluene, ethyl acetate or dimethylformamide. The base or benzoyl reactant preferably

are added to the reaction mixture with cooling. The mixture is stirred at 0°C—50°C until the reaction is substantially complete.

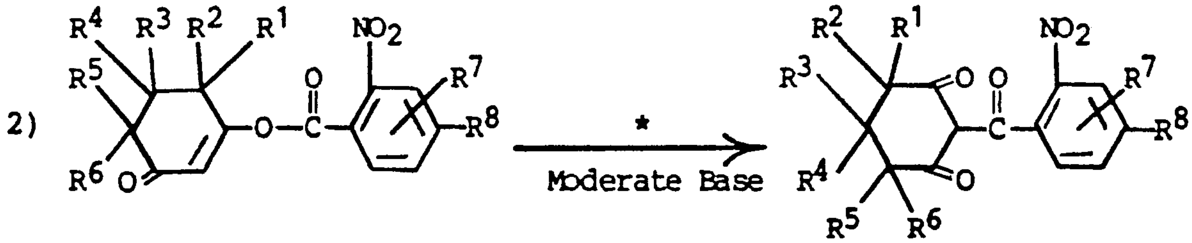


* = Cyanide source.

wherein the moderate base and $R^1$ through $R^8$ are as defined above.

Generally, in step (2) a mole of the enol ester intermediate is reacted with 1 to 4 moles of the base, preferably about 2 moles of moderate base and from 0.01 mole to about 0.5 mole or higher, preferably around 0.1 mole of the cyanide source (e.g., potassium cyanide or acetone cyanohydrin). The mixture is stirred in a reaction pot until the rearrangement is substantially complete at a temperature below 50°C, preferably about 20°C to about 40°C, and the desired product is recovered by conventional techniques.

The term "cyanide source" refers to a substance or substances which under the rearrangement conditions consists of or generates hydrogen cyanide and/or cyanide anion.

The process is conducted in the presence of a catalytic amount of a source of cyanide anion and/or hydrogen cyanide, together with a molar excess, with respect to the enol ester, of a moderate base.

Preferred cyanide sources are alkali metal cyanides such as sodium and potassium cyanide; cyanohydrins of methyl alkyl ketones having from 1—4 carbon atoms in the alkyl groups, such as acetone or methyl isobutyl ketone cyanohydrins; cyanohydrins of benzaldehyde or of $C_2$—$C_5$ aliphatic aldehydes such as acetaldehyde, propionaldehyde, etc., cyanohydrins; zinc cyanide; tri(lower alkyl) silyl cyanides, notably trimethyl silyl cyanide; and hydrogen cyanide itself. Hydrogen cyanide is considered most advantageous as it produces relatively rapid reaction and is inexpensive. Among cyanohydrins the preferred cyanide source is acetone cyanohydrin.

The cyanide source is used in an amount up to about 50 mole percent based on the enol ester. It may be used in as little as about 1 mole percent to produce an acceptable rate of reaction at about 40°C on a small scale. Larger scale reactions give more reproducible results with slightly higher catalyst levels of about 2 mole percent. Generally about 1—10 mole % of the cyanide source is preferred.

The process is conducted with a molar excess, with respect to the enol ester, of a moderate base. By the term "moderate base" is meant a substance which acts as a base yet whose strength or activity as a base lies between that of strong bases such as hydroxides (which could cause hydrolysis of the enol ester) and that of weak bases such as bicarbonates (which would not function effectively). Moderate bases suitable for use in this embodiment include both organic bases such as tertiary amines and inorganic bases such as alkali metal carbonates and phosphates. Suitable tertiary amines include trialkylamines such as triethylamine, trialkanolamines such as triethanolamine, and pyridine. Suitable inorganic bases include potassium carbonate and trisodium phosphate.

The base is used in an amount of from about 1 to about 4 moles per mole of enol ester, preferably about 2 moles per mole.

When the cyanide source is an alkali metal cyanide, particularly potassium cyanide, a phase transfer catalyst may be included in the reaction. Particularly suitable phase transfer catalysts are the Crown ethers.

A number of different solvents may be usable in this process, depending on the nature of the acid chloride or the acylated product. A preferred solvent for this reaction is 1,2-dichloroethane. Other solvents which may be employed, depending on the reactants or products include toluene, acetonitrile, methylene chloride, ethyl acetate, dimethylformamide, and methyl isobutyl ketone (MIBK).

In general, depending on the nature of the reactants and the cyanide source, the rearrangement may be conducted at temperatures up to about 50°C.

The above described substituted benzoyl chlorides can be prepared from the corresponding substituted benzoic acids according to the teaching of *Reagents for Organic Synthesis, Vol. I,* L. F. Fieser and M. Fieser, pp. 767—769 (1967).

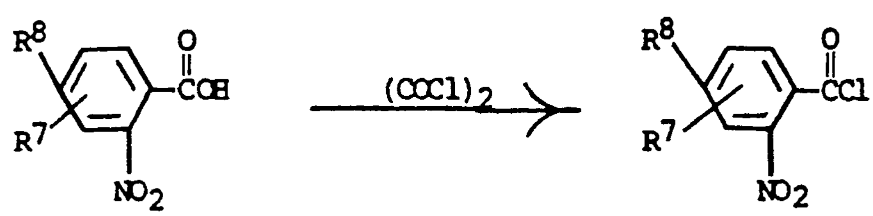


wherein $R^7$ and $R^8$ are as previously defined.

The substituted benzoic acids can be prepared by a wide variety of general methods according to the teaching of *The Chemistry of Carboxylic Acids and Esters,* S. Patai, editor, J. Wiley and Sons, New York, N.Y. (1969) and *Survey of Organic Synthesis,* C. A. Buehler and D. F. Pearson, J. Wiley and Sons, (1970).

The following are three representative examples of the methods described therein.

a) 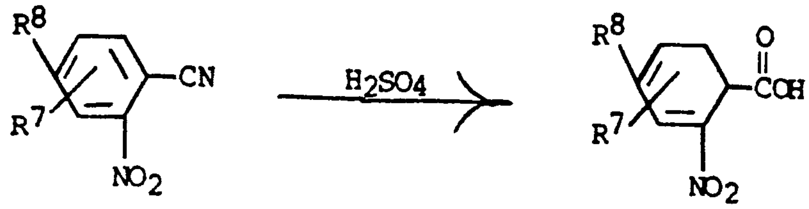


wherein $R^7$ and $R^8$ are as previously defined.

In reaction (a) the substituted benzonitrile is heated to reflux in aqueous sulfuric acid for several hours. The mixture is cooled and the reaction product is isolated by conventional techniques.

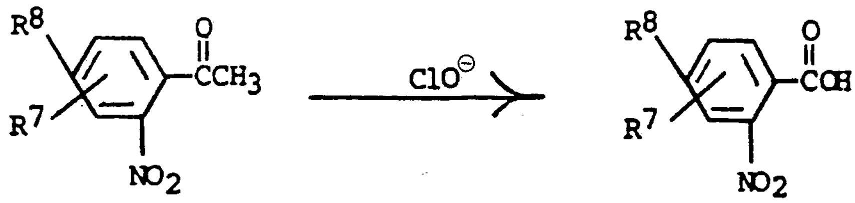


wherein $R^7$ and $R^8$ are as previously defined.

In reaction (b) the substituted acetophenone is heated to reflux for several hours in an aqueous hypochlorite solution. The mixture is cooled and the reaction product is isolated by conventional techniques.

c) 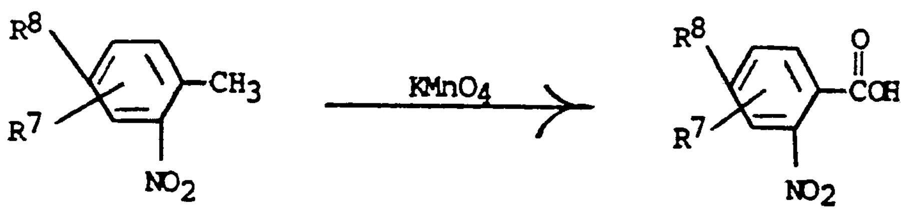


wherein $R^7$ and $R^8$ are as previously defined.

In reaction (c) the substituted toluene is heated to reflux in an aqueous solution of potassium permanganate for several hours. The solution is then filtered and the reaction product is isolated by conventional techniques.

The following examples teach the synthesis of representative compounds of this invention.

Example 1

2-(2'-Nitrobenzoyl)-1,3-cyclohexanedione

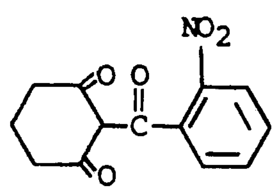

2-Nitrobenzoyl chloride (5.0 g, 27 mmol) and cyclohexanedione (3.0 g, 27 mmol) were dissolved in methylene chloride. Triethylamine (4.9 ml, 35 mmol) was added dropwise and the resulting solution stirred for one hour. The solution was washed with 2 normal hydrochloric acid (2N HCl), water, 5% potassium carbonate solution and saturated sodium chloride solution, dried over anhydrous magnesium sulfate ($MgSO_4$) and concentrated under vacuum. The residue was dissolved in 20 ml acetonitrile. Triethylamine (1 equivalent) and potassium cyanide (40 mol %) were added and the solution stirred for one hour at room temperature. After dilution with ether, the solution was washed with 2N HCl and extracted with 5% potassium carbonate solution. The aqueous extract was acidified and ether was added. Filtration of the resulting mixture yielded 3.2 g of the desired compound (m.p. 132—135°C) which was identified by nuclear magnetic resonance spectroscopy, infrared spectroscopy and mass spectroscopy.

Example 2
2-(2'-Nitrobenzoyl)-5,5-dimethyl-1,3-cyclohexanedione

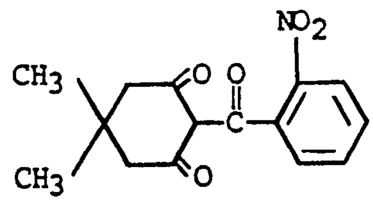

Triethylamine (3.4 ml, 25 mmol) was added dropwise to a methylene chloride solution of 2-nitrobenzoyl chloride (3.5 g, 19 mmol) and 5,5-dimethylcyclohexanedione (2.4 g, 19 mmol). After stirring for one hour at room temperature an additional 3 equivalents of triethylamine and 0.4 ml acetone cyanohydrin were added. The solution was stirred for 2.5 hours, then washed with 2N HCl and extracted with 5% potassium carbonate solution. The basic extracts were acidified with 2N HCl and extracted with ether. The ether portion was washed with saturated sodium chloride solution, dried over anhydrous magnesium sulfate and concentrated under vacuum. The residue was recrystallized from ethyl acetate yielding 2.0 g of the desired compound (m.p. 130—133°C) which was identified as such by nuclear magnetic resonance spectroscopy, infrared spectroscopy and mass spectroscopy.

The following is a table of certain selected compounds that are preparable according to the procedure described hereto. Compound numbers are assigned to each compound and are used throughout the remainder of the application.

TABLE I

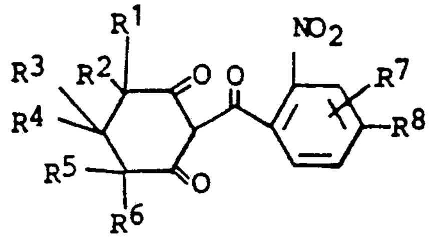

| Comp. No. | $R^1$ | $R^2$ | $R^3$ | $R^4$ | $R^5$ | $R^6$ | $R^7$ | $R^8$ | $n_D^{30}$ or m.p. |
|---|---|---|---|---|---|---|---|---|---|
| 1 | $CH_3$ | H | H | H | H | H | H | H | viscous oil |
| 2 | $CH_3$ | $CH_3$ | H | H | $CH_3$ | H | H | H | viscous oil |
| 3[a)] | H | H | H | H | H | H | H | H | 132-135 |
| 4 | $CH_3$ | $CH_3$ | H | H | H | H | H | H | viscous oil |
| 5[b)] | H | H | $CH_3$ | $CH_3$ | H | H | H | H | 130-133 |
| 6 | $CH_3$ | H | H | H | $CH_3$ | H | H | H | viscous oil |
| 7 | $CH_3$ | $CH_3$ | H | H | H | H | H | $CF_3$ | 52-61 |
| 8 | H | H | H | H | H | H | H | $CF_3$ | 88-94 |
| 9 | H | H | $CH_3$ | $CH_3$ | H | H | H | $CF_3$ | 89-97 |
| 10 | $CH_3$ | $CH_3$ | H | H | H | H | 3-$CH_3$ | H | 119-122 |
| 11 | $CH_3$ | $CH_3$ | H | H | H | H | 3-Cl | H | 72-79 |
| 12 | $CH_3$ | $CH_3$ | H | H | H | H | H | Cl | 118-121 |
| 13 | $CH_3$ | $CH_3$ | H | H | H | H | 5-Cl | H | 118-120 |
| 14 | $CH_3$ | $CH_3$ | H | H | H | H | 5-F | H | 130-133 |
| 15 | $CH_3$ | $CH_3$ | H | H | H | H | 3-$CH_3O$ | H | 139-142 |
| 16 | $CH_3$ | $CH_3$ | $CH_3$ | H | H | H | H | $CF_3$ | viscous oil |
| 17 | $CH_3$ | $CH_3$ | H | H | H | H | H | $NO_2$ | viscous oil |
| 18 | $CH_3$ | $CH_3$ | H | H | H | H | H | Br | viscous oil |
| 19 | $CH_3$ | $CH_3$ | H | H | H | $CH_3$ | 5-$CH_3$ | H | viscous oil |
| 20 | $CH_3$ | $CH_3$ | H | H | H | H | 5-$CH_3$ | H | viscous oil |
| 21 | H | H | H | H | H | H | H | F | 123-128 |
| 22 | $CH_3$ | $CH_3$ | H | H | H | H | H | F | viscous oil |
| 23 | H | H | H | H | H | H | H | Cl | viscous oil |
| 24 | $CH_3$ | $CH_3$ | H | H | H | H | H | $SO_2CH_3$ | 130-133 |
| 25 | $CH_3$ | $CH_3$ | H | H | H | H | H | $SO_2$-n-$C_3H_7$ | viscous oil |
| 26 | H | H | H | H | H | H | H | $SO_2CH_3$ | 157-159 |
| 27 | H | H | H | H | H | H | H | $SO_2$-n-$C_3H_7$ | 120-123 |
| 28 | $CH_3$ | $CH_3$ | H | H | H | H | 5-F | H | 165-195 |

TABLE I

(continued)

| Comp. No. | $R_1$ | $R_2$ | $R_3$ | $R_4$ | $R_5$ | $R_6$ | $R_7$ | $R_8$ | $n_D^{30}$ or m.p. |
|---|---|---|---|---|---|---|---|---|---|
| 29 | $CH_3$ | $CH_3$ | H | H | H | H | H | $SO_2-C_2H_5$ | oil |
| 30 | $CH_3$ | $CH_3$ | H | H | $CH_3$ | H | H | $SO_2-CH_3$ | gum |
| 31 | $CH_3$ | n-$C_4H_9$ | H | H | H | H | H | H | viscous oil |
| 32 | H | H | i-$C_4H_9$ | H | H | H | H | H | viscous oil |
| 33 | H | H | H | H | H | H | H | $SO_2-C_2H_5$ | viscous oil |
| 34 | H | H | H | H | H | H | H | CN | viscous oil |
| 35 | H | H | H | H | H | H | H | $SO_2N(CH_3)_2$ | 158-159 |
| 36 | $CH_3$ | $CH_3$ | H | H | H | H | H | $SO_2N(CH_3)_2$ | 120-130 |
| 37 | H | H | H | H | H | H | H | $SO_2N(C_2H_5)_2$ | 158-163 |
| 38 | $CH_3$ | $CH_3$ | H | H | H | H | H | $SO_2N(C_2H_5)_2$ | oil |
| 39 | $CH_3$ | $CH_3$ | H | H | H | H | H | $CH_3$<br>/<br>$SO_2$-N<br>\\<br>n-$C_4H_9$ | oil |
| 40 | H | H | $CH_3$ | $CH_3$ | H | H | H | $SO_2-N(C_2H_5)_2$ | oil |
| 41 | H | H | H | H | H | H | H | $SC_2H_5$ | oil |
| 42 | H | H | H | H | H | H | H | S(O)-n-$C_3H_7$ | oil |
| 43 | H | H | H | H | H | H | H | S-n-$C_3H_7$ | oil |
| 44 | $CH_3$ | $CH_3$ | H | H | $CH_3$ | H | H | S-n-$C_3H_7$ | oil |
| 45 | $CH_3$ | $CH_3$ | H | H | H | H | H | S-n-$C_3H_7$ | oil |
| 46 | $CH_3$ | $CH_3$ | H | H | $CH_3$ | H | H | S-$C_2H_5$ | oil |
| 47 | $CH_3$ | $CH_3$ | H | H | H | H | H | S-$C_2H_5$ | oil |
| 48 | H | H | H | H | H | H | H | S-$CH_3$ | 94-97 |
| 49 | $CH_3$ | $CH_3$ | H | H | $CH_3$ | H | H | $CF_3$ | oil |
| 50 | $CH_3$ | $CH_3$ | H | H | H | H | H | S-$CH_3$ | oil |
| 51 | c) | H | i-$C_3H_7$ | H | H | H | H | H | 145-148 |
| 52 | $CH_3$ | $CH_3$ | H | H | H | H | 5-$CH_3O$ | Br | oil |
| 53 | H | H | $CH_3$ | $CH_3$ | H | H | H | Cl | oil |
| 54 | H | H | H | H | H | H | 3-$CH_3O$ | Cl | oil |
| 55 | $CH_3$ | $CH_3$ | H | H | H | H | 3-$CH_3O$ | Cl | oil |
| 56 | $CH_3$ | $CH_3$ | H | H | $CH_3$ | H | H | $CH_3S$ | oil |
| 57 | H | H | H | H | H | H | H | H<br>/<br>$SO_2$N<br>\\<br>n-$C_3H_7$ | 120-125 |

TABLE I

(continued)

| Comp. No. | $R_1$ | $R_2$ | $R_3$ | $R_4$ | $R_5$ | $R_6$ | $R_7$ | $R_8$ | $n_D^{30}$ or m.p. |
|---|---|---|---|---|---|---|---|---|---|
| 58 | H | H | $CH_3$ | $CH_3$ | H | H | H | CN | 175-177 |
| 59 | $CH_3$ | $CH_3$ | H | H | H | H | H | CN | 151-153 |
| 60 | $CH_3$ | $CH_3$ | H | H | $CH_3$ | H | H | CN | oil |
| 61 | c) | H | H | H | H | H | H | H | oil |
| 62 | d) | H | H | H | H | H | H | H | oil |
| 63 | H | H | $CH_3$ | H | H | H | H | Cl | 110-115 |
| 64 | H | H | $CH_3$ | H | H | H | H | $SO_2$-n-$C_3H_7$ | oil |
| 65 | d) | $CH_3$ | H | H | H | H | H | Cl | oil |
| 66 | H | H | H | H | H | H | H | $SO_2CHCl_2$ | oil |
| 67 | $CH_3$ | $CH_3$ | H | H | H | H | H | $SO_2CHCl_2$ | oil |
| 68 | H | H | H | H | H | H | c) | Br | oil |
| 69 | H | H | H | H | H | H | H | $SO_2CH_2Cl$ | oil |
| 70 | $CH_3$ | $CH_3$ | H | H | H | H | H | $SO_2CH_2Cl$ | wax |
| 71 | d) | $CH_3$ | H | H | H | H | H | H | oil |
| 72 | H | H | H | H | H | H | $C_2H_5O$ | Cl | oil |
| 73 | $CH_3$ | $CH_3$ | H | H | $CH_3$ | H | $CH_3O$ | $CF_3$ | oil |

a) Prepared in Example I.   c) = $C_2H_5OC(O)-$

b) Prepared in Example II.   d) = $i\text{-}C_3H_7OC(O)-$

Herbicidal Screening Tests

As previously mentioned, the herein described compounds produced in the above-described manner are phytotoxic compounds which are useful and valuable in controlling various plant species. Selected compounds of this invention were tested as herbicides in the following manner.

*Pre-Emergence Herbicide Test:* On the day preceding treatment, seeds of eight different weed species are planted in loamy sand soil in individual rows using one species per row across the width of a flat. The seeds used are green foxtail (FT) (*Setaria viridis*), watergrass (WG) (*Echinochloa crusgalli*), wild oat (WO) (*Avena fatua*), annual morningglory (AMG) (*Ipomoea lacunosa*), velvetleaf (VL) (*Abutilon theophrasti*), Indian mustard (MD) (*Brassica juncea*), curly dock (CD) (*Rumex crispus*), and yellow nutsedge (YNG) (*Cyperus esculentus*). Ample seeds are planted to give about 20 to 40 seedlings per row, after emergence, depending upon the size of the plants.

Using an analytical balance, 600 milligrams (mg) of the compound to be tested are weighed out on a piece of glassine weighing paper. The paper and compound are placed in a 60 milliliter (ml) wide-mouth clear bottle and dissolved in 45 ml of acetone or substituted solvent. Eighteen ml of this solution are transferred to a 60 ml wide-mouth clear bottle and diluted with 22 ml of a water and acetone mixture (19:1) containing enough polyoxyethylene sorbitan monolaurate emulsifier to give a final solution of 0.5% (v/v). The solution is then sprayed on a seeded flat on a linear spray table calibrated to deliver 80 gallons per acre (748 L/ha). The application rate is 4 lb/acre (4.48 Kg/ha).

After treatment, the flats are placed in the greenhouse at a temperature of 70 to 80°F and watered by sprinkling. Two weeks after treatment, the degree of injury or control is determined by comparison with untreated check plants of the same age. The injury rating from 0 to 100% is recorded for each species as percent control with 0% representing no injury and 100% representing complete control.

The results of the tests are shown in the following Table II.

TABLE II

Pre-Emergence Herbicidal Activity
Application Rate — 4.48 kg/ha

| Cmpd. No. | FT | WG | WO | AMG | VL | MD | CD | YNG |
|---|---|---|---|---|---|---|---|---|
| 1 | 100 | 100 | 85 | 30 | 100 | 100 | 90 | 90 |
| 2 | 100 | 100 | 100 | 50 | 100 | 100 | 95 | 95 |
| 3 | 100 | 100 | 85 | 25 | 100 | 100 | 100 | 95 |
| 4 | 100 | 100 | 100 | 20 | 100 | 85 | 95 | 90 |
| 5 | 100 | 100 | 45 | 25 | 100 | 100 | 90 | 90 |
| 6 | 100 | 100 | 95 | 40 | 100 | 100 | 85 | 90 |
| 9 | 100 | 100 | 90 | 90 | 100 | 100 | 80 | 90 |
| 10 | 100 | 90 | 20 | 10 | 100 | 70 | 100 | 90 |
| 11 | 90 | 100 | 50 | 230 | 100 | 100 | 90 | 90 |
| 12 | 100 | 100 | 95 | 80 | 100 | 100 | 90 | 90 |
| 13 | 40 | 75 | 0 | 10 | 80 | 100 | 100 | 90 |
| 14 | 50 | 0 | 0 | 0 | 100 | 80 | 70 | 90 |
| 15 | 65 | 95 | 20 | 15 | 100 | 80 | 90 | 85 |
| 17 | 100 | 100 | 60 | 30 | 100 | 100 | 90 | 35 |
| 18 | 100 | 100 | 100 | 100 | 100 | 100 | 100 | 95 |
| 19 | 100 | 100 | 0 | 50 | 100 | 100 | 100 | 95 |
| 20 | 75 | 100 | 0 | 25 | 100 | 90 | 65 | 90 |
| 21 | 100 | 100 | 100 | 80 | 100 | 100 | 90 | 95 |
| 22 | 100 | 100 | 100 | 80 | 100 | 100 | 95 | 95 |
| 23 | 100 | 100 | 98 | 95 | 100 | 100 | 100 | 95 |
| 25 | 100 | 100 | 80 | 100 | 100 | 100 | 80 | - |
| 26 | 100 | 100 | 75 | 100 | 100 | 100 | 80 | - |
| 27 | 90 | 100 | 50 | 100 | 100 | 100 | 100 | 90 |
| 28 | 75 | 50 | 50 | 0 | 100 | 100 | 90 | 65 |
| 30 | 100 | 100 | 85 | 100 | 100 | 100 | 95 | 90 |
| 31 | 85 | 75 | 0 | 25 | 100 | 25 | 0 | 35 |
| 32 | 83 | 85 | 35 | 20 | 95 | 100 | 75 | 50 |
| 36 | 100 | 100 | 50 | 100 | 100 | 100 | 100 | 75 |
| 37 | 20 | 75 | 0 | 20 | 100 | 95 | 100 | 75 |
| 38 | 85 | 95 | 40 | 60 | 100 | 100 | 75 | 85 |
| 39 | 85 | 95 | 45 | 75 | 100 | 95 | 70 | 90 |
| 51 | 60 | 60 | 35 | 0 | 25 | 0 | 0 | 30 |
| 52 | 75 | 75 | 0 | 50 | 90 | 75 | 40 | 0 |
| 65 | 100 | 100 | 80 | 100 | 100 | 100 | - | 80 |

A blank (-) indicates that the weed was not tested.

*Post-Emergence Herbicide Test:* This test is conducted in an identical manner to the testing procedure for the pre-emergence herbicide test, except the seeds of the eight different weed species are planted 10—12 days before treatment. Also, watering of the treated flats is confined to the soil surface and not to the foliage of the sprouted plants.

The results of the post-emergence herbicide test are reported in Table III.

TABLE III

Post-Emergence Herbicidal Activity
Application Rate — 4.48 kg/ha

| Cmpd. No. | FT | WG | WO | AMG | VL | MD | CD | YNG |
|---|---|---|---|---|---|---|---|---|
| 1 | 95 | 75 | 85 | 70 | 100 | 90 | 85 | 40 |
| 2 | 45 | 70 | 95 | 75 | 100 | 90 | 100 | 65 |
| 3 | 100 | 80 | 100 | 90 | - | 100 | 100 | 80 |
| 4 | 100 | 80 | 100 | 100 | - | 100 | 85 | 75 |
| 5 | 90 | 70 | 45 | 60 | 95 | 70 | 60 | 80 |
| 6 | 95 | 75 | 80 | 70 | 100 | 90 | 90 | 65 |
| 9 | 100 | 90 | 90 | 100 | 100 | 100 | 95 | 85 |
| 10 | 45 | 75 | 10 | 15 | 100 | 100 | 20 | 75 |
| 11 | 100 | 70 | 60 | 75 | 100 | 100 | 100 | 45 |
| 12 | 100 | 75 | 100 | 100 | 100 | 100 | 90 | 90 |
| 13 | 30 | 55 | 0 | 30 | 60 | 60 | 15 | 60 |
| 14 | 20 | 65 | 0 | 40 | 70 | 60 | 40 | 25 |
| 15 | 20 | 75 | 30 | 20 | 100 | 70 | 60 | 40 |
| 17 | 85 | 80 | 50 | 65 | 95 | 95 | 100 | 60 |
| 18 | 100 | 95 | 100 | 100 | 100 | 100 | 100 | 75 |
| 19 | 20 | 95 | 30 | 100 | 100 | 35 | 30 | 70 |
| 20 | 30 | 80 | 15 | 100 | 100 | 45 | 20 | 70 |
| 21 | 100 | 80 | 100 | 55 | 100 | 90 | 100 | 80 |
| 22 | 100 | 80 | 100 | 60 | 100 | 95 | 95 | 95 |
| 23 | 100 | 90 | 90 | 100 | 100 | 100 | 85 | 70 |
| 25 | 70 | 75 | 50 | 85 | 90 | 85 | 60 | 75 |
| 26 | 100 | 85 | 85 | 95 | 95 | 95 | 90 | 60 |
| 27 | 90 | 90 | 60 | 100 | 100 | 100 | 100 | - |
| 28 | 15 | 45 | 20 | 50 | 75 | 80 | 15 | 30 |
| 30 | 100 | 100 | 80 | 85 | 85 | 85 | 100 | - |
| 31 | 80 | 90 | 100 | 100 | 100 | 100 | 100 | 60 |
| 32 | 75 | 85 | 85 | 75 | 75 | 90 | 95 | 50 |
| 36 | 35 | 50 | 35 | 70 | 50 | 50 | 35 | 60 |
| 37 | 60 | 75 | 15 | 70 | 70 | 90 | 90 | 40 |
| 38 | 95 | 90 | 65 | 70 | 90 | 90 | 100 | 50 |
| 39 | 95 | 85 | 30 | 50 | 70 | 80 | 100 | 50 |
| 51 | 60 | 75 | 60 | 35 | 30 | 60 | 40 | 60 |
| 52 | 60 | 75 | 25 | 100 | 100 | 100 | 100 | 75 |
| 65 | 70 | 50 | 70 | 90 | 80 | 85 | - | 80 |

A blank (-) indicates the weed was not tested.

Pre-Emergence Multi-Weed Herbicide Test: Several compounds were evaluated at an application rate of 2, 1, 1/2 or 1/4 lb/acre (2.24, 1.12, 0.56 or 0.28 kg/ha) for pre-emergence activity against a larger number of weed species.

The process was generally similar to the pre-emergence herbicide test described above except that only 300, 150, 75 or 37.5 milligrams of test compound were weighed out and the application rate was 40 gallons per acre.

Redroot pigweed (PW) and curly dock (CD) were eliminated in this test and the following weed species were added:

| | | | |
|---|---|---|---|
| *Grasses:* | downy brome | *Bromus tectorum* | (DB) |
| | annual ryegrass | *Lolium multiflorum* | (ARG) |
| | shattercane | *Sorghum bicolor* | (SHC) |
| | hemp sesbania | *Sesbania exaltata* | (SESB) |
| | sickepod | *Cassia obtusifolia* | (SP) |
| | cocklebur | *Xanthium sp.* | (CB) |
| | broadleaf signalgrass | *Brachiaria platyphylla* | (BSG) |

The results of the test are shown in Tables IV, V and VI.

TABLE IV

Pre-Emergence Multi-weed Herbicide Test

Application Rate - 2.24 kg/ha

| Cmpd. No. | DB | FT | ARG | WG | SHC | WO | BSG | AMG | SESB | VL | SP | MD | YNG | CB |
|---|---|---|---|---|---|---|---|---|---|---|---|---|---|---|
| 7 | 100 | 100 | 100 | 100 | 100 | 100 | 100 | 100 | 100 | 100 | 100 | 100 | 95 | 100 |
| 8 | 100 | 100 | 100 | 100 | 100 | 100 | 100 | 100 | 100 | 100 | 100 | 100 | 95 | - |
| 16[a] | 70 | 100 | 65 | 100 | 100 | 60 | 98 | 55 | 100 | 100 | 90 | 100 | 90 | - |
| 24 | 100 | 100 | 100 | 100 | 100 | 100 | 100 | 100 | 100 | 100 | 100 | 100 | 100 | - |
| 29 | 100 | 100 | 100 | 100 | 100 | 100 | 100 | 100 | 100 | 100 | 30 | 100 | 95 | 80 |
| 33 | 75 | 15 | 60 | 90 | 90 | 20 | 95 | 100 | 100 | 100 | 60 | 100 | 95 | 100 |
| 53 | 100 | 100 | 100 | 100 | 100 | 90 | 100 | 100 | 100 | 100 | 100 | 100 | 95 | 100 |
| 57 | 100 | 100 | 25 | 100 | 100 | 30 | 25 | 100 | 100 | 100 | 100 | 100 | 95 | 100 |
| 64[a] | - | 0 | 0 | 95 | 35 | 0 | 15 | 50 | 75 | 75 | 25 | - | 75 | 40 |
| 66[a] | - | 0 | 15 | 15 | 50 | 20 | 50 | 100 | 100 | 75 | 0 | - | 90 | 100 |
| 67[a] | - | 0 | 0 | 100 | 100 | 0 | 25 | 95 | 75 | 50 | 25 | - | 30 | 75 |
| 69[a] | - | 30 | 0 | 100 | 100 | 0 | 70 | 100 | 100 | 100 | 35 | - | 95 | 100 |
| 70[a] | - | 100 | 10 | 100 | 100 | 25 | 65 | 100 | 100 | 100 | 0 | - | 95 | 100 |

(-) = Not tested.

(a) = Tested at 0.28 kg/ha.

TABLE V

Pre-Emergence Multi-weed Herbicide Test

Application Rate - 1.12 kg/ha

| Cmpd. No. | DB | FT | ARG | WG | SHC | WO | BSG | AMG | SESB | VL | SP | MD | YNG | CB |
|---|---|---|---|---|---|---|---|---|---|---|---|---|---|---|
| 34 | 90 | 85 | 30 | 95 | - | 45 | 98 | 75 | 100 | 100 | 40 | 100 | 50 | - |
| 35 | 100 | 85 | 70 | 100 | - | 90 | 100 | 100 | 100 | 100 | 40 | 100 | 75 | - |
| 40 | 100 | 100 | 20 | 100 | - | 70 | 100 | 98 | 98 | 100 | 20 | 100 | 50 | - |
| 41 | 100 | 100 | 80 | 100 | - | 60 | 100 | 100 | 100 | 100 | 25 | 100 | 95 | - |
| 42 | 50 | 60 | 40 | 85 | - | 30 | 100 | 100 | 100 | 100 | 100 | 100 | 90 | - |
| 43 | 90 | 95 | 60 | 100 | - | 30 | 98 | 100 | 98 | 100 | 45 | 100 | 95 | - |
| 44 | 60 | 100 | 20 | 100 | - | 60 | 100 | 100 | 90 | 100 | 20 | 100 | 80 | - |
| 45 | 95 | 100 | 35 | 100 | - | 60 | 90 | 100 | 100 | 100 | 0 | 100 | 90 | - |
| 46 | 100 | 100 | 90 | 100 | - | 95 | 100 | 100 | 100 | 100 | 40 | 100 | 95 | - |
| 47 | 100 | 100 | 100 | 100 | - | 98 | 100 | 100 | 98 | 100 | 30 | 100 | 95 | - |
| 48 | 100 | 100 | 100 | 100 | - | 100 | 100 | 100 | 100 | 100 | 90 | 100 | 100 | - |
| 49 | 100 | 100 | 100 | 100 | - | 100 | 100 | 100 | 100 | 100 | 90 | 100 | - | - |
| 50 | 100 | 100 | 100 | 100 | 100 | 85 | 100 | 100 | 100 | 100 | 90 | 100 | 98 | - |
| 54 | 100 | 100 | 85 | 100 | 100 | 15 | 100 | 25 | 100 | 100 | 65 | 100 | 95 | 100 |
| 55 | 85 | 100 | 35 | 100 | 98 | 15 | 100 | 15 | 100 | 100 | 65 | 100 | 95 | - |
| 56 | 100 | 100 | 100 | 100 | 100 | 100 | 100 | 100 | 100 | 100 | 100 | 100 | 100 | 100 |
| 58 | 98 | 100 | 40 | 95 | 40 | 20 | 95 | 100 | 100 | 100 | 85 | 100 | 100 | 95 |
| 59 | 100 | 100 | 100 | 100 | 100 | 90 | 100 | 100 | 100 | 100 | 100 | 100 | 85 | 80 |
| 60 | 100 | 100 | 100 | 100 | 100 | 100 | 100 | 100 | 100 | 100 | 75 | 100 | 85 | 80 |

(-) = Not tested.

TABLE VI

Pre-Emergence Multi-weed Herbicide Test

Application Rate - 0.56 kg/ha

| Cmpd. No. | DB | FT | ARG | WG | SHC | WO | BSG | AMG | SESB | VL | SP | MD | YNG | CB |
|---|---|---|---|---|---|---|---|---|---|---|---|---|---|---|
| 61 | - | 100 | 65 | 100 | 65 | 20 | 80 | 20 | 40 | 80 | 10 | - | 20 | 0 |
| 62 | - | 50 | 35 | 70 | 50 | 0 | 0 | 0 | 25 | 50 | 0 | - | 0 | 0 |
| 63 | 100 | 100 | 100 | 100 | 100 | 95 | 100 | 90 | 95 | 100 | 75 | 100 | 100 | 85 |
| 68 | - | 0 | 20 | 0 | 0 | 0 | 0 | 60 | 100 | 100 | 90 | - | 75 | 75 |
| 71 | - | 50 | 40 | 50 | 75 | 40 | 35 | 75 | 50 | 70 | 0 | - | 75 | 75 |
| 72 | - | 35 | 60 | 100 | 85 | 50 | 100 | 25 | 65 | 100 | 35 | - | 100 | 35 |
| 73 | - | 90 | 70 | 100 | 95 | 25 | 0 | 70 | 100 | 100 | 0 | - | 50 | 25 |

(-) = Not tested.

*Post-Emergence Multi-Weed Herbicide Test:* This test is conducted in an identical manner to the testing procedure for the post-emergence herbicide test, except the seeds of the eight weed species used in the pre-emergence multi-weed herbicide test were used and the seeds were planted 10—12 days before treatment. Also, watering of the treated flats is confined to the soil surface and not to the foliage of the sprouted plants.

The results of the post-emergence multi-weed herbicide test are reported in Tables VII, VIII and IX.

TABLE VII

Post-Emergence Multi-Weed Herbicidal Activity
Application Rate — 2.24 kg/ha

| Cmpd. No. | DB | FT | ARG | WG | SHC | WO | BSG | AMG | SESB | VL | SP | MD | YNG | CB |
|---|---|---|---|---|---|---|---|---|---|---|---|---|---|---|
| 7 | 100 | 100 | 100 | 100 | 80 | 90 | 10 | 95 | 100 | 100 | 55 | 100 | 45 | 100 |
| 16[a] | 100 | 85 | 35 | 100 | 100 | 100 | 100 | 100 | 100 | 100 | 100 | 100 | 85 | 70 |
| 24 | 100 | 100 | 100 | 100 | 100 | 100 | 100 | 100 | 100 | 100 | 100 | 100 | 100 | - |
| 29 | 100 | 100 | 60 | 100 | 90 | 100 | 100 | 100 | 100 | 100 | 100 | 100 | 100 | - |
| 33 | 90 | 98 | 85 | 100 | 100 | 80 | 100 | 100 | 100 | 100 | 90 | 100 | 90 | 100 |
| 53 | 100 | 100 | 60 | 100 | 100 | 100 | 100 | 100 | 100 | 100 | 100 | 100 | 100 | - |
| 57 | 25 | 40 | 10 | 100 | 10 | 0 | 10 | 100 | 95 | 100 | 35 | 100 | - | 100 |
| 64[a] | - | 0 | 0 | 90 | 0 | 0 | 85 | 40 | 100 | 80 | 50 | - | 35 | 100 |
| 66[a] | - | 0 | 0 | 65 | 0 | 0 | 70 | 75 | 80 | 75 | 0 | - | 25 | 75 |
| 67[a] | - | 0 | 0 | 75 | 35 | 0 | 40 | 70 | 80 | 60 | 0 | - | 0 | 100 |
| 69[a] | - | 0 | 0 | 80 | 35 | 0 | 100 | 90 | 100 | 100 | 40 | - | 35 | 100 |
| 70[a] | - | 100 | 0 | 100 | 70 | 90 | 90 | 100 | 100 | 100 | 30 | - | 25 | 100 |

(-) = Not tested.
(a) = Tested at 0.28 kg/ha.

TABLE VIII

Post-Emergence Multi-weed Herbicide Test
Application Rate - 1.12 kg/ha

| Cmpd. No. | DB | FT | ARG | WG | SHC | WO | BSG | AMG | SESB | VL | SP | MD | YNG | CB |
|---|---|---|---|---|---|---|---|---|---|---|---|---|---|---|
| 34 | 90 | 85 | 30 | 95 | - | 45 | 98 | 75 | 100 | 100 | 40 | 100 | 50 | - |
| 35 | 100 | 85 | 70 | 100 | - | 90 | 100 | 100 | 100 | 100 | 40 | 100 | 75 | - |
| 40 | 75 | 100 | 5 | 100 | - | 50 | 75 | 100 | 100 | 100 | 40 | 100 | 30 | - |
| 41- | - | - | - | - | - | - | - | - | - | - | - | - | - | - |
| 42 | 40 | 100 | 35 | 100 | - | 50 | 80 | 100 | 100 | 100 | 80 | 100 | 70 | - |
| 43 | 60 | 70 | 20 | 100 | - | 55 | 60 | 100 | 100 | 100 | 95 | 100 | 70 | - |
| 44 | - | - | - | - | - | - | - | - | - | - | - | - | - | - |
| 45 | - | - | - | - | - | - | - | - | - | - | - | - | - | - |
| 46 | - | - | - | - | - | - | - | - | - | - | - | - | - | - |
| 47 | 90 | 100 | 35 | 100 | - | 75 | 90 | 100 | 100 | 100 | 25 | 100 | 45 | - |
| 48 | 80 | 100 | 60 | 100 | - | 60 | 80 | 100 | 100 | 100 | 85 | 100 | 60 | - |
| 49 | - | - | - | - | - | - | - | - | - | - | - | - | - | - |
| 50 | 90 | 80 | 60 | 95 | 80 | 90 | 100 | 100 | 100 | 100 | 65 | 100 | 80 | - |
| 54 | 35 | 50 | 30 | 100 | 30 | 15 | 90 | 100 | 100 | 100 | 100 | 100 | 95 | - |
| 55 | 100 | 100 | 20 | 100 | 90 | 20 | 100 | 90 | 100 | 100 | 100 | 100 | - | - |
| 56 | 75 | 90 | 75 | 95 | 90 | 25 | 100 | 100 | 100 | 100 | 90 | 100 | 90 | 100 |
| 58 | 70 | 100 | 40 | 100 | 95 | 30 | 95 | 100 | 95 | 100 | 95 | 100 | 95 | 85 |
| 59 | 90 | 100 | 95 | 100 | 100 | 50 | 100 | 100 | 100 | 100 | 95 | 100 | 100 | 95 |
| 60 | 95 | 100 | 100 | 100 | 100 | 75 | 95 | 100 | 100 | 100 | 95 | 100 | 100 | 100 |

(-) = Not tested.

TABLE IX

Post-Emergence Multi-weed Herbicide Test

Application Rate - 0.56 kg/ha

| Cmpd. No. | DB | FT | ARG | WG | SHC | WO | BSG | AMG | SESB | VL | SP | MD | YNG | CB |
|---|---|---|---|---|---|---|---|---|---|---|---|---|---|---|
| 61 | - | 40 | 0 | 50 | 35 | 0 | 40 | 75 | 95 | 100 | 0 | - | 25 | 50 |
| 62 | - | 35 | 0 | 20 | 0 | 0 | 20 | 35 | 60 | 100 | 0 | - | 0 | 100 |
| 63 | 100 | 100 | 85 | 98 | 85 | 100 | 100 | 100 | 100 | 100 | 100 | 85 | 85 | 95 |
| 68 | - | 30 | 40 | 85 | 0 | 25 | 60 | 80 | 95 | 100 | 95 | - | 0 | 80 |
| 71 | - | 50 | 0 | 80 | 65 | 0 | 75 | 100 | 80 | 100 | 50 | - | 25 | 100 |
| 72 | - | 90 | 70 | 80 | 50 | 50 | 75 | 85 | 100 | 100 | 90 | - | 100 | 100 |
| 73 | - | 100 | 15 | 100 | 75 | 75 | 85 | 100 | 100 | 90 | 60 | - | 80 | 100 |

(-) = Not tested.

The compounds of the present invention are useful as herbicides and can be applied in a variety of ways at various concentrations. In practice, the compounds herein defined are formulated into herbicidal compositions, by admixture, in herbicidally effective amounts, with the adjuvants and carriers normally employed for facilitating the dispersion of active ingredients for agricultural applications, recognizing the fact that the formulation and mode of application of a toxicant may affect the activity of the materials in a given application. Thus, these active herbicide compounds may be formulated as granules of relatively large particle size, as wettable powders, as emulsifiable concentrates, as powdery dusts, as solutions or as any of several other known types of formulations, depending upon the desired mode of application. Preferred formulations for pre-emergence herbicidal applications are wettable powders, emulsifiable concentrates and granules. These formulations may contain as little as about 0.5% to as much as about 95% or more by weight of active ingredient. A herbicidally effective amount depends upon the nature of the seeds or plants to be controlled and the rate of application varies from about 0.05 to approximately 25 pounds per acre, preferably from about 0.1 to about 10 pounds per acre.

Wettable powders are in the form of finely divided particles which disperse readily in water or other dispersants. The wettable powder is ultimately applied to the soil either as a dry dust or as a dispersion in water or other liquid. Typical carriers for wettable powders include fuller's earth, kaolin clays, silicas and other readily wet organic or inorganic diluents. Wettable powders normally are prepared to contain about 5% to about 95% of the active ingredient and usually also contain a small amount of wetting, dispersing, or emulsifying agent to facilitate wetting and dispersion.

Emulsifiable concentrates are homogeneous liquid compositions which are dispersible in water or other dispersant, and may consist entirely of the active compound with a liquid or solid emulsifying agent, or may also contain a liquid carrier, such as xylene, heavy aromatic naphthal, isophorone and other nonvolatile organic solvents. For herbicidal application, these concentrates are dispersed in water or other liquid carrier and normally applied as a spray to the area to be treated. The percentage by weight of the essential active ingredient may vary according to the manner in which the composition is to be applied, but in general comprises about 0.5% to 95% of active ingredient by weight of the herbicidal composition.

Granular formulations wherein the toxicant is carried on relatively coarse particles, are usually applied without dilution to the area in which suppression of vegetation is desired. Typical carriers for granular formulations include sand, fuller's earth, bentonite clays, vermiculite, perlite and other organic or inorganic materials which absorb or which may be coated with the toxicant. Granular formulations normally are prepared to contain about 5% to about 25% of active ingredients which may include surface-active agents such heavy aromatic naphthas, kerosene or other petroleum fractions, or vegetable oils; and/or stickers such as destrins, glue or synthetic resins.

Typical wetting, dispersing or emulsifying agents used in agricultural formulations include, for example, the alkyl and alkylaryl sulfonates and sulfates and their sodium salts; polyhydric alcohols; and other types of surface-active agents, many of which are available in commerce. The surface-active agent, when used, normally comprises from 0.1% to 15% by weight of the herbicidal composition.

Dusts, which are free-flowing admixtures of the active ingredient with finely divided solids such as talc, clays, flours and other organic and inorganic solids which act as dispersants and carriers for the toxicant, are useful formulations for soil-incorporating application.

Pastes, which are homogeneous suspensions of a finely divided solid toxicant in a liquid carrier such as water or oil, are employed for specific purposes. These formulations normally contain about 5% to about 95% of active ingredient by weight, and may also contain small amounts of a wetting, dispersing or emulsifying agent to facilitate dispersion. For application, the pastes are normally diluted and applied as a spray to the area to be affected.

Other useful formulations for herbicidal applications include simple solutions of the active ingredient in a dispersant in which it is completely soluble at the desired concentration, such as acetone, alkylated naphthalenes, xylene and other organic solvents. Pressurized sprays, typically aerosols, wherein the active ingredient is dispersed in finely-divided form as a result of vaporization of a low boiling dispersant solvent carrier, such as the Freons, may also be used.

The phytotoxic compositions of this invention are applied to the plants in the conventional manner. Thus, the dust and liquid compositions can be applied to the plant by the use of power-dusters, boom and hand sprayers and spray dusters. The compositions can also be applied from airplanes as a dust or a spray because they are effective in very low dosages. In order to modify or control growth of germinating seeds or emerging seedlings, as a typical example, the dust and liquid compositions are applied to the soil according to conventional methods and are distributed in the soil to a depth of at least 1/2 inch below the soil surface. It is not necessary that the phytotoxic compositions be admixed with the soil particles since these compositions can also be applied merely by spraying or sprinkling the surface of the soil. The phytotoxic compositions of this invention can also be applied by addition to irrigation water supplied to the field to be treated. This method of application permits the penetration of the compositions into the soil as the water is absorbed therein. Dust compositions, granular compositions or liquid formulations applied to the surface of the soil can be distributed below the surface of the soil by conventional means such as discing, dragging or mixing operations.

EMULSIFIABLE CONCENTRATE FORMULATIONS

| General Formula with Ranges | | Specific Formula | |
|---|---|---|---|
| Herbicidal compound | 5—55 | herbicidal compound | 54 |
| surfactant(s) | 5—25 | proprietory blend of oil-soluble sulfonates polyoxyethylene ethers | 10 |
| solvent(s) | 20—90 | polar solvent | 27 |
| | | petroleum hydrocarbon | 9 |
| | 100% | | 100% |

WETTABLE POWDER FORMULATIONS

| | | | |
|---|---|---|---|
| herbicidal compound | 3—90 | herbicidal compound | 80 |
| wetting agent | 0.5—2 | sodium dialkyl naphthalene sulfonate | 0.5 |
| dispersing agent | 1—8 | sodium lignosulfonate | 7 |
| diluent(s) | 8.5—87 | attapulgite clay | 12.5 |
| | 100% | | 100% |

EXTRUDED GRANULAR FORMULATIONS

| | | | |
|---|---|---|---|
| herbicidal compound | 1—20 | herbicidal compound | 10 |
| binding agent | 0—10 | lignin sulfonate | 5 |
| diluent(s) | 70—99 | calcium carbonate | 85 |
| | 100% | | 100% |

FLOWABLE FORMULATIONS

| | | | |
|---|---|---|---|
| herbicidal compound | 20—70 | herbicidal compound | 45 |
| surfactant(s) | 1—10 | polyoxyethylene ether | 5 |
| suspending agent(s) | 0.05—1 | attagel | 0.05 |
| antifreeze agent | 1—10 | propylene glycol | 10 |
| antimicrobial agent | 1—10 | BIT | 0.03 |
| antifoam agent | 0.1—1 | silicone defoamer | 0.02 |
| solvent | 7.95—77.85 | water | 39.9 |
| | 100% | | 100% |

The phytotoxic compositions of this invention can also contain other additaments, for example, fertilizers and other herbicides, pesticides and the like, used as adjuvant or in combination with any of the above-described adjuvants. Other phytotoxic compounds useful in combination with the above-described compounds include, for example, anilides such as 2-benzothiazole-2-yloxy-N-methyl acetanilide, 2-chloro-2′,6′-dimethyl-N-(n-propylethyl) acetanilide, 2-chloro-2′,6′-diethyl-N-(butoxymethyl) acetanilide; 2,4-dichlorophenoxyacetic acids, 2,4,5-trichlorophenoxyacetic acid, 2-methyl-4-chlorophenoxyacetic acid and the salts, esters and amides thereof; triazine derivatives, such as 2,4-bis(3-methoxypropylamino)-6-methylthio-s-triazine, 2-chloro-4-ethylamino-6-isopropylamino-s-triazine, and 2-ethylamino-4-isopropyl-amino-6-methyl-mercapto-s-triazine; urea derivatives, such as 3-(3,5-dichlorophenyl)-1,1-dimethylurea and 3-(p-chlorophenyl)-1,1-dimethylurea; and acetamides such as N,N-diallyl-α-chloroacetamide, and the like; benzoic acids such as 3-amino-2,5-dichlorobenzoic acid; thiocarbamates such as S-(1,1-dimethylbenzyl)-piperidene-1-carbothioste, 3-(4-chlorophenyl)-methyl diethylcarbothioate, ethyl-1-hexahydro-1,4-azepine-1-carbothioate, S-ethyl-hexahydro-1H-azepine-1-carbothioate, S-propyl N,N-dipropylthiocarbamate, S-ethyl N,N-dipropylthiocarbamate, S-ethyl cyclohexylethylthiocarbamate and the like; anilines such as 4-(methylsulfonyl)-2,6-dinitro-N,N-substituted aniline, 4-trifluoromethyl-2,6-dinitro-N,N-dipn-propyl aniline, 4-trifluoromethyl-2,6-dinitro-N-ethyl-N-butyl aniline, 2-[4-(2,4-dichlorophenoxy)phenoxy]propanoic acid, 2-[1-(ethoxyimino)butyl]-5-[2-ethylthio)propyl]-3-hydroxy-2-cyclohexene-1-one, (±)-butyl-2[4-[(5-trifluoromethyl)-2-pyridinyl)oxy]phenoxy]propanate, sodium 5-[2-chloro-4-(trifluoromethyl)phenoxy]-2-nitrobenzoate, 3-isopropyl-1H-2,1,3-benzothiadiazine-4(3H)-one-2,2-dioxide, and 4-amino-6-tert-butyl-3(methylthio)-as-triazin-5(4H)-one or 4-amino-6-(1,1-dimethylethyl)-3-(methylthio)-1,2,4-triazin-5(4H)-one and S-(O,O-diisopropyl)-benzene sulfonamide. Fertilizers useful in combination with the active ingredients include, for example, ammonium nitrate, urea and superphosphate. Other useful additaments include materials in which plant organisms take root and grow such as compost, manure, humus, sand, and the like.

**Claims for the Contracting States: BE CH DE FR GB IT LI NL SE**

1. A compound of the formula

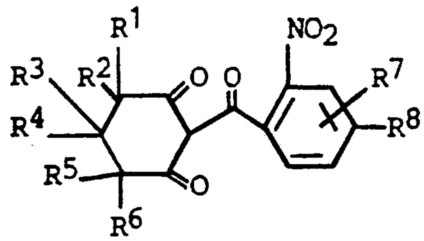

wherein

$R^1$ is hydrogen or $C_1$—$C_4$ alkyl;

$R^2$ is hydrogen, $C_1$—$C_4$ alkyl or

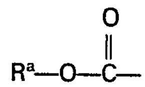

wherein $R^a$ is $C_1$—$C_4$ alkyl; or

$R^1$ and $R^2$ together are alkylene having 3 to 6 carbon atoms;

$R^3$ is hydrogen or $C_1$—$C_4$ alkyl;

$R^4$ is hydrogen or $C_1$—$C_4$ alkyl;

$R^5$ is hydrogen or $C_1$—$C_4$ alkyl;

$R^6$ is hydrogen or $C_1$—$C_4$ alkyl; and

$R^7$ and $R^8$ independently are (1) hydrogen; (2) halogen; (3) $C_1$—$C_4$ alkyl; (4) $C_1$—$C_4$ alkoxy; (5) $OCF_3$; (6) cyano; (7) nitro; (8) $C_1$—$C_4$ haloalkyl; (9) $R^bSO_n$— wherein n is the integer 0, 1 or 2; and $R^b$ is (a) $C_1$—$C_4$ alkyl; (b) $C_1$—$C_4$ alkyl substituted with halogen or cyano; (c) phenyl; or (d) benzyl; (10) —$NR^cR^d$ wherein $R^c$ and $R^d$ independently are hydrogen or $C_1$—$C_4$ alkyl; (11) $R^eC(O)$— wherein $R^e$ is $C_1$—$C_4$ alkyl or $C_1$—$C_4$ alkoxy; or (12) $SO_2NR^cR^d$ wherein $R^c$ and $R^d$ are as defined, with the proviso that $R^7$ is not attached to the 6-position.

2. The compounds of Claim 1 wherein $R^1$ is hydrogen or methyl; $R^2$ is hydrogen or methyl; $R^3$ is hydrogen or methyl; $R^4$ is hydrogen or methyl; $R^5$ is hydrogen or methyl; $R^6$ is hydrogen or methyl; $R^7$ and $R^8$ independently are (1) hydrogen; (2) halogen; (3) $C_1$—$C_4$ alkyl; (4) $C_1$—$C_4$ alkoxy; (5) $OCF_3$; (6) cyano; (7) nitro; (8) $C_1$—$C_4$ haloalkyl; (9) $R^bSO_n$— wherein n is the integer 0, 1 or 2; and $R^b$ is (a) $C_1$—$C_4$ alkyl; (b) $C_1$—$C_4$ alkyl substituted with halogen or cyano; (c) phenyl; or (d) benzyl; (10) —$NR^cR^d$ wherein $R^c$ and $R^d$ independently are hydrogen or $C_1$—$C_4$ alkyl; (11) $R^eC(O)$— wherein $R^e$ is $C_1$—$C_4$ alkyl or $C_1$—$C_4$ alkoxy; or (12) $SO_2NR^cR^d$ wherein $R^c$ and $R^d$ are as defined.

3. The compound of Claim 2 wherein $R^7$ and $R^8$ are independently are hydrogen; chlorine; fluorine; bromine; methyl; methoxy; $OCF_3$; cyano; nitro; trifluoromethyl; $R^bSO_n$— wherein n is the integer 2 and $R^b$ is methyl, chloromethyl, trifluoromethyl, cyanomethyl, ethyl, or n-propyl; —$NR^cR^d$ wherein $R^c$ and $R^d$ independently are hydrogen or $C_1$—$C_4$ alkyl; $R^eC(O)$— where $R^e$ is $C_1$—$C_4$ alkyl or $C_1$—$C_4$ alkoxy or $SO_2NR^cR^d$ wherein $R^c$ and $R^d$ are as defined and $R^7$ is in the 3-position.

4. The compound of Claim 2 wherein $R^7$ is hydrogen and $R^8$ is hydrogen, chlorine, bromine, fluorine, $CF_3$ or $R^bSO_2$ wherein $R^b$ is $C_1$—$C_4$ alkyl or $C_1$—$C_4$ haloalkyl.

5. The compound of Claim 2 wherein $R^1$ is methyl; $R^2$ is methyl; $R^3$ is hydrogen; $R^4$ is hydrogen; $R^5$ is

hydrogen; $R^6$ is hydrogen; $R^7$ is hydrogen; and $R^8$ is hydrogen.

6. The compound of Claim 2 wherein $R^1$ is methyl; $R^2$ is methyl; $R^3$ is hydrogen; $R^4$ is hydrogen; $R^5$ is methyl; $R^6$ is hydrogen; $R^7$ is hydrogen; and $R^8$ is hydrogen.

7. The compound of Claim 2 wherein $R^1$ is methyl; $R^2$ is methyl; $R^3$ is hydrogen; $R^4$ is hydrogen; $R^5$ is hydrogen; $R^6$ is hydrogen; $R^7$ is hydrogen; and $R^8$ is trifluoromethyl.

8. The compound of Claim 2 wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen; and $R^8$ is trifluoromethyl.

9. The compound of Claim 2 wherein $R^1$ is methyl; $R^2$ is methyl; $R^3$ is hydrogen; $R^4$ is hydrogen; $R^5$ is hydrogen; $R^6$ is hydrogen; $R^7$ is hydrogen; and $R^8$ is chlorine.

10. The compound of Claim 2 wherein $R^1$ is methyl; $R^2$ is methyl; $R^3$ is hydrogen; $R^4$ is hydrogen; $R^5$ is hydrogen; $R^6$ is hydrogen; $R^7$ is hydrogen; and $R^8$ is fluorine.

11. The compound of Claim 2 wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen; and $R^8$ is chlorine.

12. The compound of Claim 2 wherein $R^1$ is methyl; $R^2$ is methyl; $R^3$ is hydrogen; $R^4$ is hydrogen; $R^5$ is hydrogen; $R^6$ is hydrogen; $R^7$ is hydrogen; and $R^8$ is $CH_3SO_2$—.

13. The compound of Claim 2 wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen; and $R^8$ is n-$C_3H_7SO_2$.

14. The compound of Claim 2 wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen; and $R^8$ is $CH_3SO_2$.

15. The compound of Claim 2 wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen; and $R^8$ is $C_2H_5SO_2$.

16. The compounds of Claim 2 wherein $R^7$ is hydrogen.

17. The compounds of Claim 3 wherein $R^7$ is hydrogen.

18. The compound of Claim 2 wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen; and $R^8$ is cyano.

19. The compound of Claim 1 wherein $R^1$ and $R^2$ are hydrogen or both methyl.

20. The compound of Claim 19 wherein $R^8$ is $—SO_2CH_3$.

21. The compound of Claim 19 wherein $R^8$ is $—SO_2CH_2Cl$.

22. The compound of Claim 1 wherein $R^8$ is $CF_3$.

23. The compound of Claim 1 wherein $R^8$ is $—SO_2CH_3$.

24. The compound of Claim 1 wherein $R^8$ is chlorine.

25. The compound of Claim 1 wherein $R^8$ is $—SO_2CH_2Cl$.

26. The compound of Claim 1 wherein $R^8$ is $—SO_2$-n-$C_3H_7$.

27. The method of controlling undesirable vegetation comprising applying to the area where control is desired, an herbicidally effective amount of a compound described in Claims 1—26.

28. An herbicidal composition comprising an herbicidally active 2-(2-nitrobenzoyl)-1,3-cyclohexanedione and an inert carrier therefor.

29. The herbicidal composition of Claim 28 wherein the 2-(2-nitrobenzoyl)-1,3-cyclohexanedione is a compound of Claims 1—26.

30. The method of controlling undesirable vegetation comprising applying to the area where control is desired, an herbicidal composition comprising an herbicidally active 2-(2-nitrobenzoyl)-1,3-cyclohexanedione and an inert carrier therefor.

31. The method of Claim 30 wherein the 2-(2-nitrobenzoyl)-1,3-cyclohexanedione has a $C_1$—$C_4$ alkylsulfonyl or $C_1$—$C_4$ haloalkylsulfonyl substitution on the phenyl ring.

32. The method of Claim 31 wherein said alkylsulfonyl or haloalkylsulfonyl substitution is at the 4-position of the phenyl ring.

33. The herbicidal composition of Claim 28 wherein the 2-(2-nitrobenzoyl)-1,3-cyclohexanedione has a $C_1$—$C_4$ alkylsulfonyl or $C_1$—$C_4$ haloalkylsulfonyl substitution on the phenyl ring.

34. The herbicidal composition of Claim 33 wherein said alkylsulfonyl or haloalkylsulfonyl substitution is at the 4-position of the phenyl ring.

35. The method of Claim 30 wherein the 2-(2-nitrobenzoyl)-1,3-cyclohexanedione has a $C_1$—$C_4$ haloalkyl substitution on the phenyl ring.

36. The method of Claim 30 wherein said haloalkyl substitution is at the 4-position on the phenyl ring.

37. The herbicidal composition of Claim 28 wherein the 2-(2-nitrobenzoyl)-1,3-cyclohexanedione has a $C_1$—$C_4$ haloalkyl substitution on the phenyl ring.

38. The herbicidal composition of Claim 37 wherein said haloalkyl substitution is at the 4-position of the phenyl ring.

39. The herbicidal composition of Claim 37 wherein said haloalkyl is $CF_3$.

40. The method of Claim 35 wherein said haloalkyl is $CF_3$.

41. The method of Claim 27 wherein $R^1$ and $R^2$ are hydrogen or both methyl.

42. The method of Claim 41 wherein $R^8$ is $—SO_2CH_3$, $—SO_2CH_2Cl$, $CF_3$, $—SO_2CH_3$, chlorine, $—SO_2CH_2Cl$ or $—SO_2$-n-$C_3H_7$.

43. The composition of matter of Claim 28 wherein $R^1$ and $R^2$ are hydrogen or both methyl.

44. The composition of matter of Claim 43 wherein $R^8$ is $—SO_2CH_3$, $—SO_2CH_2Cl$, $CF_3$, $—SO_2CH_3$, chlorine, $—SO_2CH_2Cl$ or $—SO_2$—n—$C_3H_7$.

45. A process for preparing a compound of the formula

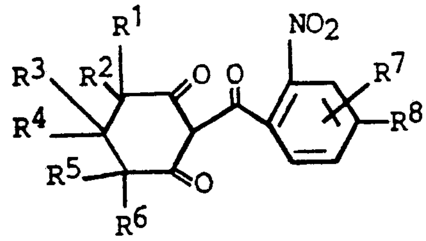

wherein

$R^1$ is hydrogen or $C_1$—$C_4$ alkyl;

$R^2$ is hydrogen, $C_1$—$C_4$ alkyl or

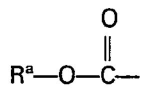

wherein $R^a$ is $C_1$—$C_4$ alkyl; or

$R^1$ and $R^2$ together are alkylene having 3 to 6 carbon atoms;

$R^3$ is hydrogen or $C_1$—$C_4$ alkyl;

$R^4$ is hydrogen or $C_1$—$C_4$ alkyl;

$R^5$ is hydrogen or $C_1$—$C_4$ alkyl;

$R^6$ is hydrogen or $C_1$—$C_4$ alkyl; and

$R^7$ and $R^8$ independently are (1) hydrogen; (2) halogen; (3) $C_1$—$C_4$ alkyl; (4) $C_1$—$C_4$ alkoxy; (5) $OCF_3$; (6) cyano; (7) nitro; (8) $C_1$—$C_4$ haloalkyl; (9) $R^bSO_n$— wherein n is the integer 0, 1 or 2; and $R^b$ is (a) $C_1$—$C_4$ alkyl; (b) $C_1$—$C_4$ alkyl substituted with halogen or cyano; (c) phenyl; or (d) benzyl; (10) —$NR^cR^d$ wherein $R^c$ and $R^d$ independently are hydrogen or $C_1$—$C_4$ alkyl; (11) $R^eC(O)$— wherein $R^e$ is $C_1$—$C_4$ alkyl or $C_1$—$C_4$ alkoxy; or (12) $SO_2NR^cR^d$ wherein $R^c$ and $R^d$ are as defined, with the proviso that $R^7$ is not attached to the 6-position comprising

(a) reacting a dione of the formula

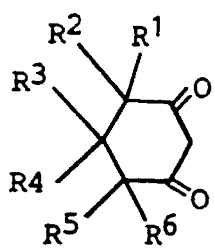

wherein $R^1$ through $R^6$ are as defined with a substituted benzoyl reactant of the formula

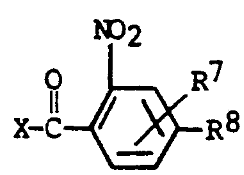

wherein $R^7$ and $R^8$ are as defined and X is halogen, $C_1$—$C_4$ alkyl-C(O)—O—, $C_1$—$C_4$ alkoxy-C(O)—O— or

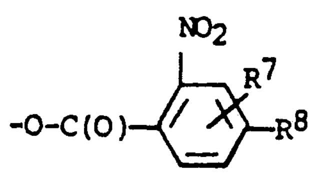

wherein $R^7$ and $R^8$ in this portion of the molecule are identical with those in the reactant shown above with at least a mole of a moderate base to form an enol ester of the formula

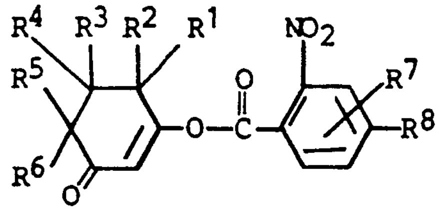

wherein $R^1$ through $R^8$ are as defined and in step (2) reacting a mole of the enol ester intermediate with 1 to 4 moles of a moderate base, and from 0.01 mole to about 0.5 mole or higher of a cyanide source to form a compound of the formula

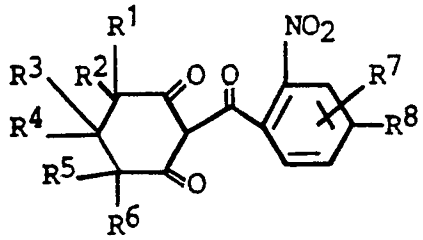

wherein $R^1$ through $R^8$ are as defined above.

46. The process of Claim 45 wherein X is halogen, the moderate base is tri-$C_1$—$C_6$ alkylamine, pyridine, alkali metal carbonate or alkali metal phosphate and the cyanide source alkali metal cyanide, cyanohydrins of methyl $C_1$—$C_4$ alkyl ketones, cyanohydrins of benzaldehyde or $C_2$—$C_5$ aliphatic aldehydes; cyanohydrins, zinc cyanide; tri(lower alkyl) silyl cyanides or hydrogen cyanide.

47. The process of Claim 46 wherein X is chlorine, the moderate base is tri-$C_1$—$C_6$ alkylamine, pyridine, sodium carbonate or sodium phosphate and the cyanide source is potassium cyanide, acetone cyanohydrin or hydrogen cyanide.

**Claims for the Contracting State: AT**

1. A process for preparing a compound of the formula

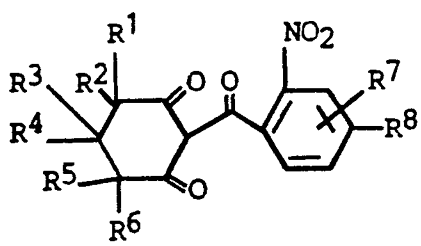

wherein

$R^1$ is hydrogen or $C_1$—$C_4$ alkyl;

$R^2$ is hydrogen, $C_1$—$C_4$ alkyl or

$$R^a\text{—O—}\overset{\overset{\displaystyle O}{\|}}{C}\text{—}$$

wherein $R^a$ is $C_1$—$C_4$ alkyl; or

$R^1$ and $R^2$ together are alkylene having 3 to 6 carbon atoms;

$R^3$ is hydrogen or $C_1$—$C_4$ alkyl;

$R^4$ is hydrogen or $C_1$—$C_4$ alkyl;

$R^5$ is hydrogen or $C_1$—$C_4$ alkyl;

$R^6$ is hydrogen or $C_1$—$C_4$ alkyl; and

$R^7$ and $R^8$ independently are (1) hydrogen; (2) halogen; (3) $C_1$—$C_4$ alkyl; (4) $C_1$—$C_4$ alkoxy; (5) $OCF_3$; (6) cyano; (7) nitro; (8) $C_1$—$C_4$ haloalkyl; (9) $R^bSO_n$— wherein n is the integer 0, 1 or 2; and $R^b$ is (a) $C_1$—$C_4$ alkyl; (b) $C_1$—$C_4$ alkyl substituted with halogen or cyano; (c) phenyl; or (d) benzyl; (10) —$NR^cR^d$ wherein $R^c$ and $R^d$ independently are hydrogen or $C_1$—$C_4$ alkyl; (11) $R^eC(O)$— wherein $R^e$ is $C_1$—$C_4$ alkyl or $C_1$—$C_4$ alkoxy; or (12) $SO_2NR^cR^d$ wherein $R^c$ and $R^d$ are as defined, with the proviso that $R^7$ is not attached to the 6-position comprising

(a) reacting a dione of the formula

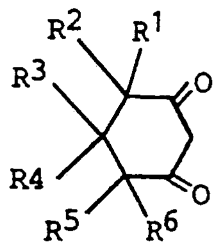

wherein $R^1$ through $R^6$ are as defined with a substituted benzoyl reactant of the formula

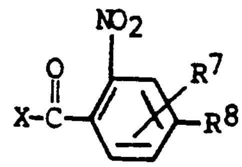

wherein $R^7$ and $R^8$ are as defined and X is halogen, $C_1$—$C_4$ alkyl-C(O)—O—, $C_1$—$C_4$ alkoxy-C(O)—O— or

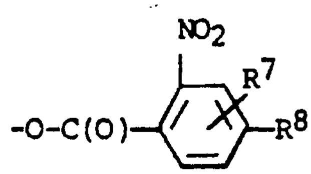

wherein $R^7$ and $R^8$ in this portion of the molecule are identical with those in the reactant shown above with at least a mole of a moderate base to form an enol ester of the formula

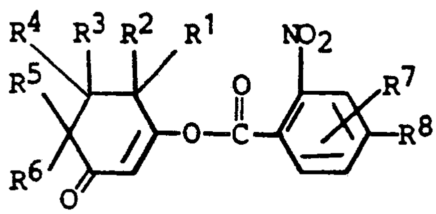

wherein $R^1$ through $R^8$ are as defined and in step (2) reacting a mole of the enol ester intermediate with 1 to 4 moles of a moderate base, and from 0.01 mole to about 0.5 mole or higher of a cyanide source to form a compound of the formula

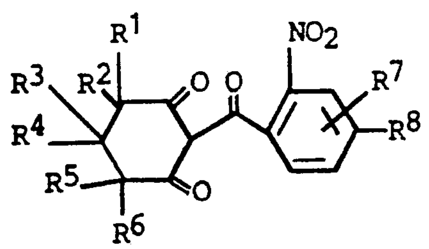

wherein $R^1$ through $R^8$ are as defined above.

2. The process of Claim 1 wherein X is halogen, the moderate base is tri-$C_1$—$C_6$ alkylamine, pyridine, alkali metal carbonate or alkali metal phosphate and the cyanide source alkali metal cyanide, cyanohydrins of methyl $C_1$—$C_4$ alkyl ketones, cyanohydrins of benzaldehyde or $C_2$—$C_5$ aliphatic aldehydes; cyanohydrins, zinc cyanide; tri(lower alkyl) silyl cyanides or hydrogen cyanide.

3. The process of Claim 2 wherein X is chlorine, the moderate base is tri-$C_1$—$C_6$ alkylamine, pyridine, sodium carbonate or sodium phosphate and the cyanide source is potassium cyanide, acetone cyanohydrin of hydrogen cyanide.

4. The process of Claim 1 characterized in that a compound wherein $R^1$ is hydrogen or methyl; $R^2$ is hydrogen or methyl; $R^3$ is hydrogen or methyl; $R^4$ is hydrogen or methyl; $R^5$ is hydrogen or methyl; $R^6$ is

hydrogen or methyl; $R^7$ and $R^8$ independently are (1) hydrogen; (2) halogen; (3) $C_1$—$C_4$ alkyl; (4) $C_1$—$C_4$ alkoxy; (5) $OCF_3$; (6) cyano; (7) nitro; (8) $C_1$—$C_4$ haloalkyl; (9) $R^bSO_n$— wherein n is the integer 0, 1 or 2; and $R^b$ is (a) $C_1$—$C_4$ alkyl; (b) $C_1$—$C_4$ alkyl substituted with halogen or cyano; (c) phenyl; or (d) benzyl; (10) —$NR^cR^d$ wherein $R^c$ and $R^d$ independently are hydrogen or $C_1$—$C_4$ alkyl; (11) $R^eC(O)$— wherein $R^e$ is $C_1$—$C_4$ alkyl or $C_1$—$C_4$ alkoxy; or (12) $SO_2NR^cR^d$ wherein $R^c$ and $R^d$ are as defined is prepared.

5. The process of Claim 1 characterized in that a compound wherein $R^7$ and $R^8$ independently are hydrogen; chlorine; fluorine; bromine; methyl; methoxy; $OCF_3$; cyano; nitro; trifluoromethyl; $R^bSO_n$— wherein n is the integer 2 and $R^b$ is methyl, chloromethyl, trifluoromethyl, cyanomethyl, ethyl, or n-propyl; —$NR^cR^d$ wherein $R^c$ and $R^d$ independently are hydrogen or $C_1$—$C_4$ alkyl; $R^eC(O)$— where $R^e$ is $C_1$—$C_4$ alkyl or $C_1$—$C_4$ alkoxy or $SO_2NR^cR^d$ wherein $R^c$ and $R^d$ are as defined and $R^7$ is in the 3-position is prepared.

6. The process of Claim 1 characterized in that a compound wherein $R^7$ is hydrogen and $R^8$ is hydrogen, chlorine, bromine, fluorine, $CF_3$ or $R^bSO_2$ wherein $R^b$ is $C_1$—$C_4$ alkyl or $C_1$—$C_4$ haloalkyl is prepared.

7. The process of Claim 1 characterized in that a compound wherein $R^1$ is methyl; $R^2$ is methyl; $R^3$ is hydrogen; $R^4$ is hydrogen; $R^5$ is hydrogen; $R^6$ is hydrogen; $R^7$ is hydrogen; and $R^8$ is hydrogen is prepared.

8. The process of Claim 1 characterized in that a compound wherein $R^1$ is methyl; $R^2$ is methyl; $R^3$ is hydrogen; $R^4$ is hydrogen; $R^5$ is methyl; $R^6$ is hydrogen; $R^7$ is hydrogen; and $R^8$ is hydrogen is prepared.

9. The process of Claim 1 characterized in that a compound wherein $R^1$ is methyl; $R^2$ is methyl; $R^3$ is hydrogen; $R^4$ is hydrogen; $R^5$ is hydrogen; $R^6$ is hydrogen; $R^7$ is hydrogen; and $R^8$ is trifluoromethyl is prepared.

10. The process of Claim 1 characterized in that a compound wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen; and $R^8$ is trifluoromethyl is prepared.

11. The process of Claim 1 characterized in that a compound wherein $R^1$ is methyl; $R^2$ is methyl; $R^3$ is hydrogen; $R^4$ is hydrogen; $R^5$ is hydrogen; $R^6$ is hydrogen; $R^7$ is hydrogen; and $R^8$ is chlorine is prepared.

12. The process of Claim 1 characterized in that a compound wherein $R^1$ is methyl; $R^2$ is methyl; $R^3$ is hydrogen; $R^4$ is hydrogen; $R^5$ is hydrogen; $R^6$ is hydrogen; $R^7$ is hydrogen; and $R^8$ is fluorine is prepared.

13. The process of Claim 1 characterized in that a compound wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen; and $R^8$ is chlorine is prepared.

14. The process of Claim 1 characterized in that a compound wherein $R^1$ is methyl; $R^2$ is methyl; $R^3$ is hydrogen; $R^4$ is hydrogen; $R^5$ is hydrogen; $R^6$ is hydrogen; $R^7$ is hydrogen; and $R^8$ is $CH_3SO_2$— is prepared.

15. The process of Claim 1 characterized in that a compound wherein $R^1$ is hydrogen $R^2$ is hydrogen; $R^3$ is hydrogen; $R^4$ is hydrogen; $R^5$ is hydrogen; $R^6$ is hydrogen; $R^7$ is hydrogen; and $R^8$ is n-$C_3H_7SO_2$ is prepared.

16. The process of Claim 1 characterized in that a compound wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen; and $R^8$ is $CH_3SO_2$ is prepared.

17. The process of Claim 1 characterized in that a compound wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen; and $R^8$ is $C_2H_5SO_2$ is prepared.

18. The process of Claim 1 characterized in that a compound wherein $R^7$ is hydrogen is prepared.

19. The process of Claim 5 characterized in that a compound wherein $R^7$ is hydrogen is prepared.

20. The process of Claim 1 characterized in that a compound wherein $R^1$ is hydrogen; $R^2$ is hydrogen; $R^3$ is hydrogen; $R^4$ is hydrogen; $R^5$ is hydrogen; $R^6$ is hydrogen; $R^7$ is hydrogen and $R^8$ is cyano is prepared.

21. The process of Claim 1 characterized in that a compound wherein $R^1$ and $R^2$ are hydrogen or both methyl is prepared.

22. The process of Claim 21 characterized in that a compound wherein $R^8$ is —$SO_2CH_3$ is prepared.

23. The process of Claim 21 characterized in that a compound wherein $R^8$ is —$SO_2CH_2Cl$ is prepared.

24. The process of Claim 1 characterized in that a compound wherein $R^8$ is $CF_3$ is prepared.

25. The process of Claim 1 characterized in that a compound wherein $R^8$ is —$SO_2CH_3$ is prepared.

26. The process of Claim 1 characterized in that a compound wherein $R^8$ is chlorine is prepared.

27. The process of Claim 1 characterized in that a compound wherein $R^8$ is —$SO_2CH_2Cl$ is prepared.

28. The process of Claim 1 characterized in that a compound wherein $R^8$ is —$SO_2$-n-$C_3H_7$ is prepared.

29. The method of controlling undesirable vegetation comprising applying to the area where control is desired, a herbicidally effective amount of a compound described in Claims 1—28.

30. A process for preparing a herbicidal composition comprising mixing a herbicidally active 2-(2-nitrobenzoyl)-1,3-cyclohexanedione and an inert carrier therefor.

31. The process of Claim 30 wherein the 2-(2-nitrobenzoyl)-1,3-cyclohexanedione is a compound as prepared according to Claims 1 to 28.

32. The method of controlling undesirable vegetation comprising applying to the area where control is desired, a herbicidal composition comprising a herbicidally active 2-(2-nitrobenzoyl)-1,3-cyclohexanedione and an inert carrier therefor.

33. The method of Claim 32 wherein the 2-(2-nitrobenzoyl)-1,3-cyclohexanedione has a $C_1$—$C_4$ alkylsulfonyl or $C_1$—$C_4$ haloalkylsulfonyl substitution on the phenyl ring.

34. The method of Claim 33 wherein said alkylsulfonyl or haloalkylsulfonyl substitution is at the 4-

position of the phenyl ring.

35. The process of Claim 30 wherein the 2-(2-nitrobenzoyl)-1,3-cyclohexanedione has a $C_1$—$C_4$ alkylsulfonyl or $C_1$—$C_4$ haloalkylsulfonyl substitution on the phenyl ring.

36. The process of Claim 35 wherein said alkylsulfonyl or haloalkylsulfonyl substitution is at the 4-position of the phenyl ring.

37. The method of Claim 32 wherein the 2-(2-nitrobenzoyl)-1,3-cyclohexanedione has a $C_1$—$C_4$ haloalkyl substitution on the phenyl ring.

38. The method of Claim 32 wherein said haloalkyl substitution is at the 4-position on the phenyl ring.

39. The process of Claim 30 wherein the 2-(2-nitrobenzoyl)-1,3-cyclohexanedione has a $C_1$—$C_4$ haloalkyl substitution on the phenyl ring.

40. The process of Claim 39 wherein said haloalkyl substitution is at the 4-position of the phenyl ring.

41. The process of Claim 39 wherein said haloalkyl is $CF_3$.

42. The method of Claim 37 wherein said haloalkyl is $CF_3$.

43. The method of Claim 29 wherein $R^1$ and $R^2$ are hydrogen or both methyl.

44. The method of Claim 43 wherein $R^8$ is $—SO_2CH_3$, $—SO_2CH_2Cl$, $CF_3$, $—SO_2CH_3$, chlorine, $—SO_2CH_2Cl$ or $—SO_2$-n-$C_3H_7$.

45. The process of Claim 30 wherein $R^1$ and $R^2$ are hydrogen or both methyl.

46. The process of Claim 45 wherein $R^8$ is $—SO_2CH_3$, $—SO_2CH_2Cl$, $CF_3$, $—SO_2CH_3$, chlorine, $—SO_2CH_2Cl$ or $—SO_2$-n-$C_3H_7$.

**Patentansprüche für die Vertragsstaaten: BE CH DE FR GB IT LI NL SE**

1. Verbindung der Formel

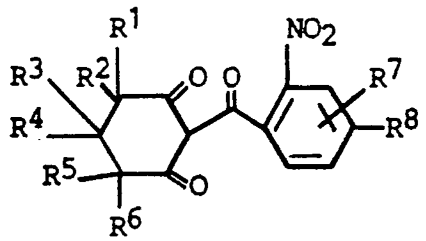

worin

$R^1$ Wasserstoff oder $C_1$—$C_4$-Alkyl bedeutet,

$R^2$ Wasserstoff, $C_1$—$C_4$-Alkyl oder

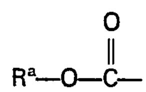

bedeutet, worin $R^a$ $C_1$—$C_4$-Alkyl bedeutet, oder worin

$R^1$ und $R^2$ zusammen Alkylen mit 3 bis 6 Kohlenstoffatomen bedeuten,

$R^3$ Wasserstoff oder $C_1$—$C_4$-Alkyl bedeutet,

$R^4$ Wasserstoff oder $C_1$—$C_4$-Alkyl bedeutet,

$R^5$ Wasserstoff oder $C_1$—$C_4$-Alkyl bedeutet,

$R^6$ Wasserstoff oder $C_1$—$C_4$-Alkyl bedeutet und

$R^7$ und $R^8$ unabhängig (1) Wasserstoff, (2) Halogen, (3) $C_1$—$C_4$-Alkyl, (4) $C_1$—$C_4$-Alkoxy, (5) $OCF_3$, (6) Cyano, (7) Nitro, (8) $C_1$—$C_4$-Haloalkyl, (9) $R^bSO_n$—, worin n eine ganze Zahl von 0, 1 oder 2 ist und $R^b$ für (a) $C_1$—$C_4$-Alkyl, (b) $C_1$—$C_4$-Alkyl, substituiert mit Halogen oder Cyano, (c) Phenyl oder (d) Benzyl steht, (10) $—NR^cR^d$, worin $R^c$ und $R^d$ unabhängig Wasserstoff oder $C_1$—$C_4$-Alkyl bedeuten, (11) $R^eC(O)$—, worin $R^e$ $C_1$—$C_4$-Alkyl oder $C_1$—$C_4$-Alkoxy bedeutet, oder (12) $SO_2NR^cR^d$, worin $R^c$ und $R^d$ die gegebenen Definitionen besitzen, bedeuten, mit der Maßgabe, daß $R^7$ nicht an die 6-Stellung gebunden ist.

2. Verbindungen nach Anspruch 1, worin $R^1$ Wasserstoff oder Methyl, $R^2$ Wasserstoff oder Methyl, $R^3$ Wasserstoff oder Methyl, $R^4$ Wasserstoff oder Methyl, $R^5$ Wasserstoff oder Methyl, $R^6$ Wasserstoff oder Methyl, $R^7$ und $R^8$ unabhängig (1) Wasserstoff, (2) Halogen, (3) $C_1$—$C_4$-Alkyl, (4) $C_1$—$C_4$-Alkoxy, (5) $OCF_3$, (6) Cyano, (7) Nitro, (8) $C_1$—$C_4$-Haloalkyl, (9) $R^bSO_n$—, worin n eine ganze Zahl von 0, 1 oder 2 ist und $R^b$ (a) $C_1$—$C_4$-Alkyl, (b) $C_1$—$C_4$-Alkyl, substituiert mit Halogen oder Cyano, (c) Phenyl oder (d) Benzyl bedeutet, (10) $—NR^cR^d$, worin $R^c$ und $R^d$ unabhängig Wasserstoff oder $C_1$—$C_4$-Alkyl bedeuten, (11) $R^eC(O)$—, worin $R^e$ $C_1$—$C_4$-Alkyl oder $C_1$—$C_4$-Alkoxy bedeutet, oder (12) $SO_2NR^cR^d$, worin $R^c$ und $R^d$ die gegebenen Definitionen besitzen, bedeuten.

3. Verbindung nach Anspruch 2, worin $R^7$ und $R^8$ unabhängig Wasserstoff, Chlor, Fluor, Brom, Methyl, Methoxy, $OCF_3$, Cyano, Nitro, Trifluormethyl, $R^bSO_n$—, worin n eine ganze Zahl von 2 ist und $R^b$ Methyl, Chlormethyl, Trifluormethyl, Cyanomethyl, Ethyl oder n-Propyl bedeutet, $—NR^cR^d$, worin $R^c$ und $R^d$ unabhängig Wasserstoff oder $C_1$—$C_4$-Alkyl bedeuten, $R^eC(O)$—, worin $R^e$ $C_1$—$C_4$-Alkyl oder $C_1$—$C_4$-Alkoxy

bedeutet, oder $SO_2NR^cR^d$, worin $R^c$ und $R^d$ die gegebenen Definitionen besitzen, bedeuten und $R^7$ in 3-Stellung vorliegt.

4. Verbindung nach Anspruch 2, worin $R^7$ Wasserstoff und $R^8$ Wasserstoff, Chlor, Brom, Fluor, $CF_3$ oder $R^bSO_2$, worin $R^b$ $C_1$—$C_4$-Alkyl oder $C_1$—$C_4$-Haloalkyl bedeutet, bedeuten.

5. Verbindung nach Anspruch 2, worin $R^1$ Methyl, $R^2$ Methyl, $R^3$ Wasserstoff, $R^4$ Wasserstoff, $R^5$ Wasserstoff, $R^6$ Wasserstoff, $R^7$ Wasserstoff und $R^8$ Wasserstoff bedeuten.

6. Verbindung nach Anspruch 2, worin $R^1$ Methyl, $R^2$ Methyl, $R^3$ Wasserstoff, $R^4$ Wasserstoff, $R^5$ Methyl, $R^6$ Wasserstoff, $R^7$ Wasserstoff und $R^8$ Wasserstoff bedeuten.

7. Verbindung nach Anspruch 2, worin $R^1$ Methyl, $R^2$ Methyl, $R^3$ Wasserstoff, $R^4$ Wasserstoff, $R^5$ Wasserstoff, $R^6$ Wasserstoff, $R^7$ Wasserstoff und $R^8$ Trifluormethyl bedeuten.

8. Verbindung nach Anspruch 2, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ Trifluormethyl bedeuten.

9. Verbindung nach Anspruch 2, worin $R^1$ Methyl, $R^2$ Methyl, $R^3$ Wasserstoff, $R^4$ Wasserstoff, $R^5$ Wasserstoff, $R^6$ Wasserstoff, $R^7$ Wasserstoff und $R^8$ Chlor bedeuten.

10. Verbindung nach Anspruch 2, worin $R^1$ Methyl, $R^2$ Methyl, $R^3$ Wasserstoff, $R^4$ Wasserstoff, $R^5$ Wasserstoff, $R^6$ Wasserstoff, $R^7$ Wasserstoff und $R^8$ Fluor bedeuten.

11. Verbindung nach Anspruch 2, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ Chlor bedeuten.

12. Verbindung nach Anspruch 2, worin $R^1$ Methyl, $R^2$ Methyl, $R^3$ Wasserstoff, $R^4$ Wasserstoff, $R^5$ Wasserstoff, $R^6$ Wasserstoff, $R^7$ Wasserstoff und $R^8$ $CH_3SO_2$— bedeuten.

13. Verbindung nach Anspruch 2, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ n-$C_3H_7SO_2$— bedeuten.

14. Verbindung nach Anspruch 2, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ $CH_3SO_2$ bedeuten.

15. Verbindung nach Anspruch 2, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ $C_2H_5SO_2$ bedeuten.

16. Verbindung nach Anspruch 2, worin $R^7$ Wasserstoff bedeutet.

17. Verbindung nach Anspruch 3, worin $R^7$ Wasserstoff, bedeutet.

18. Verbindung nach Anspruch 2, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ Cyano bedeuten.

19. Verbindung nach Anspruch 1, worin $R^1$ und $R^2$ Wasserstoff oder beide Methyl bedeuten.

20. Verbindung nach Anspruch 19, worin $R^8$ —$SO_2CH_3$ bedeutet.

21. Verbindung nach Anspruch 19, worin $R^8$ —$SO_2CH_2Cl$ bedeutet.

22. Verbindung nach Anspruch 1, worin $R^8$ $CF_3$ bedeutet.

23. Verbindung nach Anspruch 1, worin $R^8$ —$SO_2CH_3$ bedeutet.

24. Verbindung nach Anspruch 1, worin $R^8$ Chlor bedeutet.

25. Verbindung nach Anspruch 1, worin $R^8$ —$SO_2CH_2Cl$ bedeutet.

26. Verbindung nach Anspruch 1, worin $R^8$ —$SO_2$-n-$C_3H_7$ bedeutet.

27. Verfahren zur Kontrolle unerwünschter Vegetation, dadurch gekennzeichnet, daß man auf die Fläche, wo eine Kontrolle erfolgen soll, eine herbizid wirksame Menge einer Verbindung nach einem der Ansprüche 1 bis 26 anwendet.

28. Herbizide Zubereitung, dadurch gekennzeichnet, daß sie ein herbizid aktives 2-(2-Nitrobenzoyl)-1,3-cyclohexandion und einen inerten Träger dafür enthält.

29. Herbizide Zubereitung nach Anspruch 28, dadurch gekennzeichnet, daß das 2-(2-Nitrobenzoyl)-1,3-cyclohexandion eine Verbindung nach einem der Ansprüche 1 bis 26 ist.

30. Verfahren zur Kontrolle unerwünschter Vegetation, dadurch gekennzeichnet, daß man auf die Fläche, wo eine Kontrolle erfolgen soll, ein herbizides Mittel anwendet, welches ein herbizid aktives 2-(2-Nitrobenzoyl)-1,3-cyclohexandion und einen inerten Träger dafür enthält.

31. Verfahren nach Anspruch 30, dadurch gekennzeichnet, daß das 2-(2-Nitrobenzoyl)-1,3-cyclohexandion eine $C_1$—$C_4$-Alkylsulfonyl- oder $C_1$—$C_4$-Haloalkylsulfonyl-Substitution an dem Phenylring aufweist.

32. Verfahren nach Anspruch 31, dadurch gekennzeichnet, daß die Alkylsulfonyl- oder Haloalkylsulfonyl-Substitution in der 4-Stellung des Phenylrings vorhanden ist.

33. Herbizide Zubereitung nach Anspruch 28, dadurch gekennzeichnet, daß das 2-(2-Nitrobenzoyl)-1,3-cyclohexandion eine $C_1$—$C_4$-Alkylsulfonyl- oder $C_1$—$C_4$-Haloalkylsulfonyl-Substitution an dem Phenylring aufweist.

34. Herbizide Zubereitung nach Anspruch 33, dadurch gekennzeichnet, daß die Alkylsulfonyl- oder Haloalkylsulfonyl-Substitution an der 4-Stellung des Phenylrings vorhanden ist.

35. Verfahren nach Anspruch 30, dadurch gekennzeichnet, daß das 2-(2-Nitrobenzoyl)-1,3-cyclohexandion eine $C_1$—$C_4$-Haloalkyl-Substitution am Phenylring aufweist.

36. Verfahren nach Anspruch 30, dadurch gekennzeichnet, daß die Haloalkyl-Substitution in der 4-Stellung des Phenylrings vorhanden ist.

37. Herbizide Zubereitung nach Anspruch 28, dadurch gekennzeichnet, daß das 2-(2-Nitrobenzoyl)-1,3-cyclohexandion eine $C_1$—$C_4$-Haloalkyl-Substitution an dem Phenylring aufweist.

38. Herbizide Zubereitung nach Anspruch 37, dadurch gekennzeichnet, daß die Haloalkyl-Substitution

in der 4-Stellung des Phenylrings vorhanden ist.

39. Herbizide Zubereitung nach Anspruch 37, dadurch gekennzeichnet, daß das Haloalkyl $CF_3$ ist.

40. Verfahren nach Anspruch 35, dadurch gekennzeichnet, daß das Haloalkyl $CF_3$ ist.

41. Verfahren nach Anspruch 27, dadurch gekennzeichnet, daß $R^1$ und $R^2$ Wasserstoff oder beide Methyl bedeuten.

42. Verfahren nach Anspruch 41, dadurch gekennzeichnet, daß $R^8$ $—SO_2CH_3$, $—SO_2CH_2Cl$, $CF_3$, $—SO_2CH_3$, Chlor, $—SO_2CH_2Cl$ oder $SO_2$-n-$C_3H_7$ bedeutet.

43. Zubereitung nach Anspruch 28, dadurch gekennzeichnet, daß $R^1$ und $R^2$ Wasserstoff oder beide Methyl bedeuten.

44. Zubereitung nach Anspruch 43, dadurch gekennzeichnet, daß $R^8$ $—SO_2CH_3$, $—SO_2CH_2Cl$, $CF_3$, $—SO_2CH_3$, Chlor, $—SO_2CH_2Cl$ oder $—SO_2$-n-$C_3H_7$ bedeutet.

45. Verfahren zur Herstellung einer Verbindung der Formel

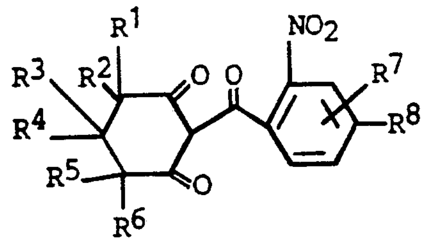

worin

$R^1$ Wasserstoff oder $C_1$—$C_4$-Alkyl bedeutet,

$R^2$ Wasserstoff, $C_1$—$C_4$-Alkyl oder

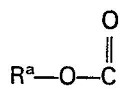

bedeutet, worin $R^a$ $C_1$—$C_4$-Alkyl bedeutet, oder worin

$R^1$ und $R^2$ zusammen Alkylen mit 3 bis 6 Kohlenstoffatomen bedeuten,

$R^3$ Wasserstoff oder $C_1$—$C_4$-Alkyl bedeutet,

$R^4$ Wasserstoff oder $C_1$—$C_4$-Alkyl bedeutet,

$R^5$ Wasserstoff oder $C_1$—$C_4$-Alkyl bedeutet,

$R^6$ Wasserstoff oder $C_1$—$C_4$-Alkyl bedeutet und

$R^7$ und $R^8$ unabhängig (1) Wasserstoff, (2) Halogen, (3) $C_1$—$C_4$-Alkyl, (4) $C_1$—$C_4$-Alkoxy, (5) $OCF_3$, (6) Cyano, (7) Nitro, (8) $C_1$—$C_4$-Haloalkyl, (9) $R^bSO_n$—, worin n eine ganze Zahl von 0, 1 oder 2 ist und $R^b$ für (a) $C_1$—$C_4$-Alkyl, (b) $C_1$—$C_4$-Alkyl, substituiert mit Halogen oder Cyano, (c) Phenyl oder (d) Benzyl steht, (10) $—NR^cR^d$, worin $R^c$ und $R^d$ unabhängig Wasserstoff oder $C_1$—$C_4$-Alkyl bedeuten, (11) $R^eC(O)$—, worin $R^e$ $C_1$—$C_4$-Alkyl oder $C_1$—$C_4$-Alkoxy bedeutet, oder (12) $SO_2NR^cR^d$, worin $R^c$ und $R^d$ die gegebenen Definitionen besitzen, bedeuten, mit der Maßgabe, daß $R^7$ nicht an die 6-Stellung gebunden ist, dadurch gekennzeichnet, daß man

(a) ein Dion der Formel

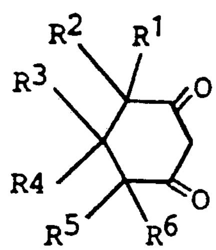

worin $R^1$ bis $R^6$ die gegebenen Definitionen besitzen, mit einen substituierten Benzoyl-Reaktionsteilnehmer der Formel

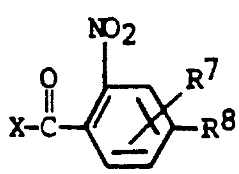

worin $R^7$ und $R^8$ die gegebenen Definitionen besitzen und X Halogen, $C_1$—$C_4$-Alkyl-C(O)—O—, $C_1$—$C_4$-Alkoxy-C(O)—O— oder

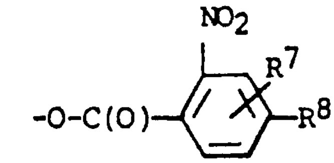

bedeutet, worin $R^7$ und $R^8$ in diesem Teil des Moleküls identisch sind mit denen in dem obigen Reaktionsteilnehmer, mit mindestens 1 Mol einer milden Base unter Bildung eines Enolesters der Formel

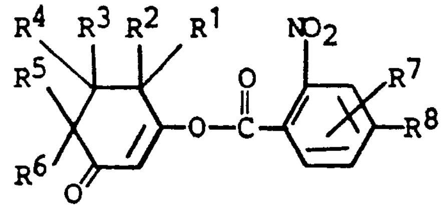

umsetzt, worin $R^1$ bis $R^8$ die oben gegebenen Definitionen besitzen, und in der Stufe (2) 1 Mol des Enolester-Zwischenprodukts mit 1 bis 4 Mol einer milden Base und von 0,01 Mol bis etwa 0,5 Mol oder mehr einer Cyanidquelle unter Bildung einer Verbindung der Formel

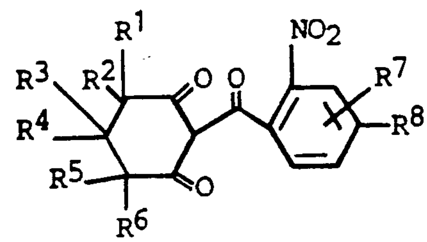

umsetzt, worin $R^1$ bis $R^8$ die oben gegebenen Definitionen besitzen.

46. Verfahren nach Anspruch 45, dadurch gekennzeichnet, daß X Halogen bedeutet, die milde Base Tri-$C_1$—$C_6$-alkylamin, Pyridin, ein Alkalimetallcarbonat oder Alkalimetallphosphat bedeutet und die Cyanidquelle ein Alkalimetallcyanid, Cyanohydrine von Methyl-$C_1$—$C_4$-alkylketonen, Cyanohydrine von Benzaldehyd oder $C_2$—$C_5$-aliphatischen-Aldehyden, Cyanohydrine, Zinkcyanid, Tri-(niedrigalkyl)-silylcyanide oder Cyanwasserstoff ist.

47. Verfahren nach Anspruch 46, dadurch gekennzeichnet, daß X Chlor bedeutet, die milde Base Tri-$C_1$—$C_6$-alkylamin, Pyridin, Natriumcarbonat oder Natriumphosphat ist und die Cyanidquelle Kaliumcyanid, Acetoncyanohydrin oder Cyanwasserstoff ist.

**Patentansprüche für den Vertragsstaat: AT**

1. Verfahren zur Herstellung einer Verbindung der Formel

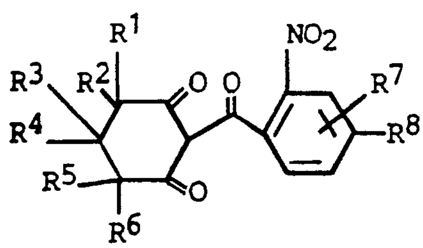

worin

$R^1$ Wasserstoff oder $C_1$—$C_4$-Alkyl bedeutet,

$R^2$ Wasserstoff, $C_1$—$C_4$-Alkyl oder

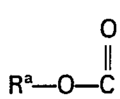

bedeutet, worin $R^a$ $C_1$—$C_4$-Alkyl bedeutet, oder worin

$R^1$ und $R^2$ zusammen Alkylen mit 3 bis 6 Kohlenstoffatomen bedeuten,

$R^3$ Wasserstoff oder $C_1$—$C_4$-Alkyl bedeutet,

$R^4$ Wasserstoff oder $C_1$—$C_4$-Alkyl bedeutet,

$R^5$ Wasserstoff oder $C_1$—$C_4$-Alkyl bedeutet,

$R^6$ Wasserstoff oder $C_1$—$C_4$-Alkyl bedeutet,

$R^7$ und $R^8$ unabhängig (1) Wasserstoff, (2) Halogen, (3) $C_1$—$C_4$-Alkyl, (4) $C_1$—$C_4$-Alkoxy, (5) $OCF_3$, (6) Cyano, (7) Nitro, (8) $C_1$—$C_4$-Haloalkyl, (9) $R^bSO_n$—, worin n eine ganze Zahl von 0, 1 oder 2 ist und $R^b$ für (a) $C_1$—$C_4$-Alkyl, (b) $C_1$—$C_4$-Alkyl, substituiert mit Halogen oder Cyano, (c) Phenyl oder (d) Benzyl steht, (10) —$NR^cR^d$, worin $R^c$ und $R^d$ unabhängig Wasserstoff oder $C_1$—$C_4$-Alkyl bedeuten, (11) $R^eC(O)$—, worin $R^e$ $C_1$—$C_4$-Alkyl oder $C_1$—$C_4$-Alkoxy bedeutet, oder (12) $SO_2NR^cR^d$, worin $R^c$ und $R^d$ die gegebenen Definitionen besitzen, bedeuten, mit der Maßgabe, daß $R^7$ nicht an die 6-Stellung gebunden ist, dadurch gekennzeichnet, daß man

(a) ein Dion der Formel

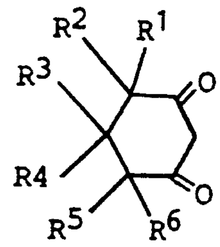

worin $R^1$ bis $R^6$ die gegebenen Definitionen besitzen, mit einem substituierten Benzoyl-Reaktionsteilnehmer der Formel

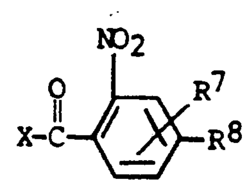

worin $R^7$ und $R^8$ die gegebenen Definitionen bezitzen und X Halogen, $C_1$—$C_4$-Alkyl-C(O)—O—, $C_1$—$C_4$-Alkoxy-C(O)—O— oder

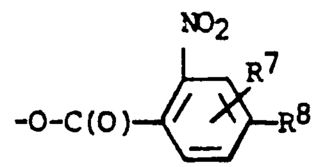

bedeutet, worin $R^7$ und $R^8$ in diesem Teil des Moleküls identisch sind mit denen in dem obigen Reaktionsteilnehmer, mit mindestens 1 Mol einer milden Base unter Bildung eines Enolesters der Formel

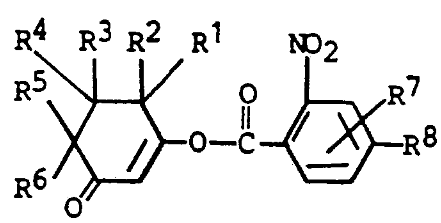

umsetzt, worin $R^1$ bis $R^8$ die oben gegebenen Definitionen besitzen, und in der Stufe (2) 1 Mol des Enolester-Zwischenprodukts mit 1 bis 4 Mol einer milden Base und von 0,01 Mol bis etwa 0,5 Mol oder mehr einer Cyanidquelle unter Bildung einer Verbindung der Formel

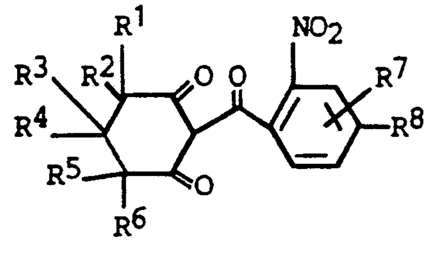

umsetzt, worin $R^1$ bis $R^8$ die oben gegebenen Definitionen besitzen.

2. Verfahren nach Anspruch 1, dadurch gekennzeichnet, daß X Halogen bedeutet, die milde Base Tri-$C_1$—$C_6$-alkylamin, Pyridin, ein Alkalimetallcarbonat oder Alkalimetallphosphat ist und die Cyanidquelle ein Alkalimetallcyanid, Cyanohdyrine von Methyl-$C_1$—$C_4$-alkylketonen, Cyanohydrine von Benzaldehyd oder $C_2$—$C_5$-aliphatischen-Aldehyden, Cyanohydrine, Zinkcyanid, Tri-(niedrigalkyl)-silylcyanide oder Cyanwasserstoff ist.

3. Verfahren nach Anspruch 2, dadurch gekennzeichnet, daß X Chlor bedeutet, die milde Base Tri-$C_1$—$C_6$-alkylamin, Pyridin, Natriumcarbonat oder Natriumphosphat ist und die Cyanidquelle Kaliumcyanid, Acetoncyanohydrin oder Cyanwasserstoff ist.

4. Verfahren nach Anspruch 1, dadurch gekennzeichnet, daß eine Verbindung hergestellt wird, worin $R^1$ Wasserstoff oder Methyl, $R^2$ Wasserstoff oder Methyl, $R^3$ Wasserstoff oder Methyl, $R^4$ Wasserstoff oder Methyl, $R^5$ Wasserstoff oder Methyl, $R^6$ Wasserstoff oder Methyl, $R^7$ und $R^8$ unabhängig (1) Wasserstoff, (2) Halogen, (3) $C_1$—$C_4$-Alkyl, (4) $C_1$—$C_4$-Alkoxy, (5) $OCF_3$, (6) Cyano, (7) Nitro, (8) $C_1$—$C_4$-Haloalkyl, (9) $R^bSO_n$—, worin n eine ganze Zahl von 0, 1 oder 2 ist und $R^b$ (a) $C_1$—$C_4$-Alkyl, (b) $C_1$—$C_4$-Alkyl, substituiert mit Halogen oder Cyano, (c) Phenyl oder (d) Benzyl bedeutet, (10) —$NR^cR^d$, worin $R^c$ und $R^d$ unabhängig Wasserstoff oder $C_1$—$C_4$-Alkyl bedeuten, (11) $R^eC(O)$—, worin $R^e$ $C_1$—$C_4$-Alkyl oder $C_1$—$C_4$-Alkoxy bedeutet, oder (12) $SO_2NR^cR^d$, worin $R^c$ und $R^d$ die gegebenen Definitionen besitzen, bedeuten.

5. Verfahren nach Anspruch 1, dadurch gekennzeichnet, daß eine Verbindung hergestellt wird, worin $R^7$ und $R^8$ unabhängig Wasserstoff, Chlor, Fluor, Brom, Methyl, Methoxy, $OCF_3$, Cyano, Nitro, Trifluormethyl, $R^bSO_n$—, worin n eine ganze Zahl von 2 ist und $R^b$ Methyl, Chlormethyl, Trifluormethyl, Cyanomethyl, Ethyl oder n-Propyl bedeutet, —$NR^cR^d$, worin $R^c$ und $R^d$ unabhängig Wasserstoff oder $C_1$—$C_4$-Alkyl bedeuten, $R^eC(O)$—, worin $R^e$ $C_1$—$C_4$-Alkyl oder $C_1$—$C_4$-Alkoxy bedeutet, oder $SO_2NR^cR^d$, worin $R^c$ und $R^d$ die oben gegebenen Definitionen besitzen, bedeuten und $R^7$ in der 3-Stellung vorhanden ist.

6. Verfahren nach Anspruch 1, dadurch gekennzeichnet, daß eine Verbindung hergestellt wird, worin $R^7$ Wasserstoff und $R^8$ Wasserstoff, Chlor, Brom, Fluor, $CF_3$ oder $R^bSO_2$ bedeuten, worin $R^b$ $C_1$—$C_4$-Alkyl oder $C_1$—$C_4$-Haloalkyl bedeutet.

7. Verfahren nach Anspruch 1, dadurch gekennzeichnet, daß eine Verbindung hergestellt wird, worin $R^1$ Methyl, $R^2$ Methyl, $R^3$ Wasserstoff, $R^4$ Wasserstoff, $R^5$ Wasserstoff, $R^6$ Wasserstoff, $R^7$ Wasserstoff und $R^8$ Wasserstoff bedeuten.

8. Verfahren nach Anspruch 1, dadurch gekennzeichnet, daß eine Verbindung hergestellt wird, worin $R^1$ Methyl, $R^2$ Methyl, $R^3$ Wasserstoff, $R^4$ Wasserstoff, $R^5$ Methyl, $R^6$ Wasserstoff, $R^7$ Wasserstoff und $R^8$ Wasserstoff bedeuten.

9. Verfahren nach Anspruch 1, dadurch gekennzeichnet, daß eine Verbindung hergestellt wird, worin $R^1$ Methyl, $R^2$ Methyl, $R^3$ Wasserstoff, $R^4$ Wasserstoff, $R^5$ Wasserstoff, $R^6$ Wasserstoff, $R^7$ Wasserstoff, und $R^8$ Trifluormethyl bedeuten.

10. Verfahren nach Anspruch 1, dadurch gekennzeichnet, daß eine Verbindung hergestellt wird, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ Trifluormethyl bedeuten.

11. Verfahren nach Anspruch 1, dadurch gekennzeichnet, daß eine Verbindung hergestellt wird, worin $R^1$ Methyl, $R^2$ Methyl, $R^3$ Wasserstoff, $R^4$ Wasserstoff, $R^5$ Wasserstoff, $R^6$ Wasserstoff, $R^7$ Wasserstoff und $R^8$ Chlor bedeuten.

12. Verfahren nach Anspruch 1, dadurch gekennzeichnet, daß eine Verbindung hergestellt wird, worin $R^1$ Methyl, $R^2$ Methyl, $R^3$ Wasserstoff, $R^4$ Wasserstoff, $R^5$ Wasserstoff, $R^6$ Wasserstoff, $R^7$ Wasserstoff und $R^8$ Fluor bedeuten.

13. Verfahren nach Anspruch 1, dadurch gekennzeichnet, daß eine Verbindung hergestellt wird, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ Chlor bedeuten.

14. Verfahren nach Anspruch 1, dadurch gekennzeichnet, daß eine Verbindung hergestellt wird, worin $R^1$ Methyl, $R^2$ Methyl, $R^3$ Wasserstoff, $R^4$ Wasserstoff, $R^5$ Wasserstoff, $R^6$ Wasserstoff, $R^7$ Wasserstoff und $R^8$ $CH_3SO_2$— bedeuten.

15. Verfahren nach Anspruch 1, dadurch gekennzeichnet, daß eine Verbindung hergestellt wird, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ $C_3H_7SO_2$ bedeuten.

16. Verfahren nach Anspruch 1, dadurch gekennzeichnet, daß eine Verbindung hergestellt wird, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ $CH_3SO_2$ bedeuten.

17. Verfahren nach Anspruch 1, dadurch gekennzeichnet, daß eine Verbindung hergestellt wird, worin $R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ $C_2H_5SO_2$ bedeuten.

18. Verfahren nach Anspruch 1, dadurch gekennzeichnet, daß eine Verbindung hergestellt wird, worin $R^7$ Wasserstoff bedeutet.

19. Verfahren nach Anspruch 5, dadurch gekennzeichnet, daß eine Verbindung hergestellt wird, worin $R^7$ Wasserstoff bedeutet.

20. Verfahren nach Anspruch 1, dadurch gekennzeichnet, daß eine Verbindung hergestellt wird, worin

$R^1$ Wasserstoff, $R^2$ Wasserstoff, $R^3$ Wasserstoff, $R^4$ Wasserstoff, $R^5$ Wasserstoff, $R^6$ Wasserstoff, $R^7$ Wasserstoff und $R^8$ Cyano bedeuten.

21. Verfahren nach Anspruch 1, dadurch gekennzeichnet, daß eine Verbindung hergestellt wird, worin $R^1$ und $R^2$ Wasserstoff oder beide Methyl bedeuten.

22. Verfahren nach Anspruch 21, dadurch gekennzeichnet, daß eine Verbindung hergestellt wird, worin $R^8$ $-SO_2CH_3$ bedeutet.

23. Verfahren nach Anspruch 21, dadurch gekennzeichnet, daß eine Verbindung hergestellt wird, worin $R^8$ $-SO_2CH_2Cl$ bedeutet.

24. Verfahren nach Anspruch 1, dadurch gekennzeichnet, daß eine Verbindung hergestellt wird, worin $R^8$ $CF_3$ bedeutet.

25. Verfahren nach Anspruch 1, dadurch gekennzeichnet, daß eine Verbindung hergestellt wird, worin $R^8$ $-SO_2CH_3$ bedeutet.

26. Verfahren nach Anspruch 1, dadurch gekennzeichnet, daß eine Verbindung hergestellt wird, worin $R^8$ Chlor bedeutet.

27. Verfahren nach Anspruch 1, dadurch gekennzeichnet, daß eine Verbindung hergestellt wird, worin $R^8$ $-SO_2CH_2Cl$ bedeutet.

28. Verfahren nach Anspruch 1, dadurch gekennzeichnet, daß eine Verbindung hergestellt wird, worin $R^8$ $-SO_2$-n-$C_3H_7$ bedeutet.

29. Verfahren zur Kontrolle unerwünschter Vegetation, dadurch gekennzeichnet, daß man auf die Fläche, wo eine Kontrolle erfolgen soll, eine herbizid wirksame Menge einer Verbindung nach einem der Ansprüche 1 bis 28 anwendet.

30. Verfahren zur Herstellung einer herbiziden Zubereitung, dadurch gekennzeichnet, daß man ein herbizid aktives 2-(2-Nitrobenzoyl)-1,3-cyclohexandion und einen inerten Träger dafür vermischt.

31. Verfahren nach Anspruch 30, dadurch gekennzeichnet, daß das 2-(2-Nitrobenzoyl)-1,3-cyclohexandion eine Verbindung ist, hergestellt gemäß den Ansprüchen 1 bis 28.

32. Verfahren zur Kontrolle unerwünschter Vegetation, dadurch gekennzeichnet, daß man auf die Fläche, wo eine Kontrolle erfolgen soll, ein herbizides Mittel anwendet, welches ein herbizid aktives 2-(2-Nitrobenzoyl)-1,3-cyclohexandion und einen inerten Träger dafür enthält.

33. Verfahren nach Anspruch 32, dadurch gekennzeichnet, daß das 2-(2-Nitrobenzoyl)-1,3-cyclohexandion eine $C_1$—$C_4$-Alkylsulfonyl- oder $C_1$—$C_4$-Haloalkylsulfonyl-Substitution an dem Phenylring aufweist.

34. Verfahren nach Anspruch 33, dadurch gekennzeichnet, daß die Haloalkylsulfonyl-Substitution in der 4-Stellung des Phenylrings vorhanden ist.

35. Verfahren nach Anspruch 30, dadurch gekennzeichnet, daß das 2-(2-Nitrobenzoyl)-1,3-cyclohexandion eine $C_1$—$C_4$-Alkylsulfonyl- oder $C_1$—$C_4$-Haloalkylsulfonyl-Substitution am Phenylring aufweist.

36. Verfahren nach Anspruch 35, dadurch gekennzeichnet, daß die Alkylsulfonyl- oder Haloalkylsulfonyl-Substitution in der 4-Stellung des Phenylrings vorhanden ist.

37. Verfahren nach Anspruch 32, dadurch gekennzeichnet, daß das 2-(2-Nitrobenzoyl)-1,3-cyclohexandion eine $C_1$—$C_4$-Haloalkyl-Substitution an dem Phenylring aufweist.

38. Verfahren nach Anspruch 32, dadurch gekennzeichnet, daß die Haloalkyl-Substitution in der 4-Stellung des Phenylrings vorhanden ist.

39. Verfahren nach Anspruch 30, dadurch gekennzeichnet, daß das 2-(2-Nitrobenzoyl)-1,3-cyclohexandion eine $C_1$—$C_4$-Haloalkyl-Substitution an dem Phenylring aufweist.

40. Verfahren nach Anspruch 39, dadurch gekennzeichnet, daß die Haloalkyl-Substitution in der 4-Stellung des Phenylrings vorhanden ist.

41. Verfahren nach Anspruch 39, dadurch gekennzeichnet, daß das Haloalkyl $CF_3$ ist.

42. Verfahren nach Anspruch 37, dadurch gekennzeichnet, daß das Haloalkyl $CF_3$ ist.

43. Verfahren nach Anspruch 29, dadurch gekennzeichnet, daß $R^1$ und $R^2$ Wasserstoff oder beide Methyl bedeuten.

44. Verfahren nach Anspruch 43, dadurch gekennzeichnet, daß $R^8$ $-SO_2CH_3$, $-SO_2CH_2Cl$, $CF_3$, $-SO_2CH_3$, Chlor, $-SO_2CH_2Cl$ oder $-SO_2$-n-$C_3H_7$ bedeutet.

45. Verfahren nach Anspruch 30, dadurch gekennzeichnet, daß $R^1$ und $R^2$ Wasserstoff oder beide Methyl bedeuten.

46. Verfahren nach Anspruch 43, dadurch gekennzeichnet, daß $R^8$ $-SO_2CH_3$, $-SO_2CH_2Cl$, $CF_3$, $-SO_2CH_3$, Chlor, $-SO_2CH_2Cl$ oder $-SO_2$-n-$C_3H_7$ bedeutet.

**Revendications pour les Etats contractants: BE CH DE FR GB IT LI NL SE**

1. Un composé de formule:

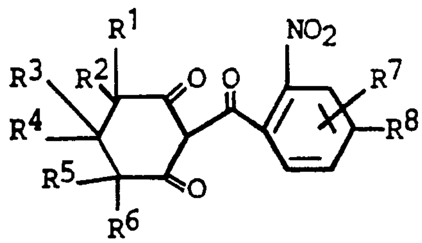

dans laquelle:

$R^1$ est un atome d'hydrogène ou un radical alkyle en $C_1$ à $C_4$;

$R^2$ est un atome d'hydrogène ou un radical alkyle en $C_1$ à $C_4$ ou un groupe $R^a$—O—C(O)— dans leque $R^a$ est un radical alkyle en $C_1$ à $C_4$; ou

$R^1$ et $R^2$, pris ensemble, forment un groupe alkylène présentant de 3 à 6 atomes de carbone;

$R^3$ est un atome d'hydrogène ou un radical alkyle en $C_1$ à $C_4$;

$R^4$ est un atome d'hydrogène ou un radical alkyle en $C_1$ à $C_4$;

$R^5$ est un atome d'hydrogène ou un radical alkyle en $C_1$ à $C_4$;

$R^6$ est un atome d'hydrogène ou un radical alkyle en $C_1$ à $C_4$; et

$R^7$ et $R^8$, indépendamment l'un de l'autre, consistent en:

(1) un atome d'hydrogène;

(2) un atome d'halogène;

(3) un radical alkyle en $C_1$ à $C_4$;

(4) un radical alkoxy en $C_1$ à $C_4$;

(5) un groupe $—OCF_3$;

(6) un groupe cyano;

(7) un groupe nitro;

(8) un groupe haloalkyle en $C_1$ à $C_4$;

(9) un groupe $R^bSO_n$— dans lequel n est un nombre entier égal à 0, 1 ou 2 et $R^b$ est:

(a) un radical alkyle en $C_1$ à $C_4$,

(b) un radical alkyle en $C_1$ à $C_4$, substitué par un atome d'halogène ou un groupe cyano;

(c) un groupe phényle; ou

(d) un groupe benzyle;

(10) un groupe $—NR^cR^d$ dans lequel $R^c$ et $R^d$, indépendamment l'un de l'autre, consistent en un atome d'hydrogène ou un radical alkyle en $C_1$ à $C_4$;

(11) un groupe $R^eC(O)$— dans lequel $R^e$ est un radical alkyle en $C_1$ à $C_4$ ou alkoxy en $C_1$ à $C_4$; ou

(12) un groupe $SO_2NR^cR^d$ dans lequel $R^c$ et $R^d$ sont tels que définis,

à condition que $R^7$ ne soit pas lié en position 6.

2. Les composés selon la revendication 1, dans lesquels $R^1$ est un atome d'hydrogène ou un radical méthyle; $R^2$ est un atome d'hydrogène ou un radical méthyle; $R^3$ est un atome d'hydrogène ou un radical méthyle; $R^4$ est un atome d'hydrogène ou un radical méthyle; $R^5$ est un atome d'hydrogène ou un radical méthyle; $R^6$ est un atome d'hydrogène ou un radical méthyle; $R^7$ et $R^8$, indépendamment l'un de l'autre, consistent en (1) un atome d'hydrogène; (2) un atome d'halogène; (3) un radical alkyle en $C_1$ à $C_4$; (4) un radical alkoxy en $C_1$ à $C_4$; (5) un groupe $OCF_3$; (6) un groupe cyano; (7) un groupe nitro; (8) un radical haloalkyle en $C_1$ à $C_4$; (9) un groupe $R^bSO_n$— dans lequel n est un nombre entier égal à 0, 1 ou 2, et $R^b$ est (a) un radical alkyle en $C_1$ à $C_4$; (b) un radical alkyle en $C_1$ à $C_4$ substitué par halogène ou cyano; (c) un groupe phényle; ou (d) un groupe benzyle; (10) un groupe $—NR^cR^d$ dans lequel $R^c$ et $R^d$ indépendamment l'un de l'autre consistent en un atome d'hydrogène ou un radical alkyle en $C_1$ à $C_4$; (11) un groupe $R^eC(O)$— dans lequel $R^e$ est un radical alkyle en $C_1$ à $C_4$ ou alkoxy en $C_1$ à $C_4$; ou (12) un groupe $SO_2NR^cR^d$ dans lequel $R^c$ et $R^d$ sont tels que définis.

3. Le composé selon la revendication 2, dans lequel $R^7$ et $R^8$ sont, indépendamment l'un de l'autre, un atome d'hydrogène, de chlore, de fluor, de brome, ou un groupe méthyle, méthoxy, $OCF_3$, cyano, nitro, trifluorométhyle; un groupe $R^bSO_n$— dans lequel n est un nombre entier égal à 2 et $R^b$ est un radical méthyle, chlorométhyle, trifluorométhyle, cyanométhyle, éthyle ou n-propyle; un groupe $—NR^cR^d$ dans lequel $R^c$ et $R^d$, indépendamment l'un de l'autre, consistent en un atome d'hydrogène ou un radical alkyle en $C_1$ à $C_4$; un groupe $R^eC(O)$— dans lequel $R^e$ est un radical alkyle en $C_1$ à $C_4$ ou alkoxy en $C_1$ à $C_4$, ou un groupe $SO_2NR^cR^d$ dans lequel $R^c$ et $R^d$ sont tels que définis ci-dessus et $R^7$ est en position 3.

4. Le composé selon la revendication 2, dans lequel $R^7$ est un atome d'hydrogène et $R^8$ est un atome d'hydrogène, de chlore, de brome, de fluor, un groupe $CF_3$ ou $R^bSO_2$ dans lequel $R^b$ est un radical alkyle en $C_1$ à $C_4$ ou haloalkyle en $C_1$ à $C_4$.

5. Le composé selon la revendication 2, dans lequel $R^1$ est un radical méthyle; $R^2$ est un radical méthyle; $R^3$ est un atome d'hydrogène; $R^4$ est un atome d'hydrogène; $R^5$ est un atome d'hydrogène; $R^6$ est

un atome d'hydrogène; $R^7$ est un atome d'hydrogène; et $R^8$ est un atome d'hydrogène.

6. Le composé selon la revendication 2, dans lequel $R^1$ est un radical méthyle; $R^2$ est un radical méthyle; $R^3$ est un atome d'hydrogène; $R^4$ est un atome d'hydrogène; $R^5$ est un radical méthyle; $R^6$ est un atome d'hydrogène; $R^7$ est un atome d'hydrogène; et $R^8$ est un atome d'hydrogène.

7. Le composé selon la revendication 2, dans lequel $R^1$ est un radical méthyle; $R^2$ est un radical méthyle; $R^3$ est un atome d'hydrogène; $R^4$ est un atome d'hydrogène; $R^5$ est un atome d'hydrogène; $R^6$ est un atome d'hyrogène; $R^7$ est un atome d'hydrogène; et $R^8$ est un radical trifluorométhyle.

8. Le composé selon la revendication 2, dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un radical trifluorométhyle.

9. Le composé selon la revendication 2, dans lequel $R^1$ est un radical méthyle; $R^2$ est un radical méthyle; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un atome de chlore.

10. Le composé selon la revendication 2, dans lequel $R^1$ est un radical méthyle; $R^2$ est un radical méthyle; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un atome de fluor.

11. Le composé selon la revendication 2, dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un atome de chlore.

12. Le composé selon la revendication 2, dans lequel $R^1$ est un radical méthyle; $R^2$ est un radical méthyle; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un groupe $CH_3SO_2$—.

13. Le composé selon la revendication 2, dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un groupe n-$C_3H_7SO_2$.

14. Le composé selon la revendication 2, dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un groupe $CH_3SO_2$.

15. Le composé selon la revendication 2, dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un groupe $C_2H_5SO_2$.

16. Les composés selon la revendication 2, dans lequel $R^7$ est un atome d'hydrogène.

17. Les composés selon la revendication 3, dans lequel $R^7$ est un atome d'hydrogène.

18. Le composé selon la revendication 2, dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un groupe cyano.

19. Le composé selon la revendication 1, dans lequel $R^1$ et $R^2$ sont des atomes d'hydrogène ou tous les deux des radicaux méthyles.

20. Le composé selon la revendication 19, dans lequel $R^8$ est un groupe $-SO_2CH_3$.

21. Le composé selon la revendication 19, dans lequel $R^8$ est le groupe $-SO_2CH_2Cl$.

22. Le composé selon la revendication 1, dans lequel $R^8$ est le groupe $CF_3$.

23. Le composé selon la revendication 1, dans lequel $R^8$ est le groupe $-SO_2CH_3$.

24. Le composé selon la revendication 1, dans lequel $R^8$ est un atome de chlore.

25. Le composé selon la revendication 1, dans lequel $R^8$ est le groupe $-SO_2CH_2Cl$.

26. Le composé selon la revendication 1, dans lequel $R^8$ est le groupe $-SO_2$-n-$C_3H_7$.

27. Procédé pour contrôler la végétation indésirable, consistant à appliquer aux zones où l'on souhaite effectuer le contrôle une quantité efficace du point de vue herbicide d'un composé selon l'une quelconque des revendications 1 à 26.

28. Une composition herbicide comprenant une quantité active du point de vue herbicide de 2-(2-nitrobenzoyl)-1,3-cyclohexanedione et un support ou véhicule inerte pour ce composé.

29. La composition herbicide selon la revendication 28, dans laquelle la 2-(2-nitrobenzoyl)-1,3-cyclohexanedione est un composé selon les revendications 1 à 26.

30. Le procédé pour contrôler la végétation indésirable, consistant à appliquer aux zones où le contrôle est souhaité une composition herbicide comprenant une 2-(2-nitrobenzoyl)-1,3-cyclohexanedione active dû point de vue herbicide et un support ou véhicule inerte pour ce composé.

31. Le procédé selon la revendication 30, dans lequel la 2-(2-nitrobenzoyl)-1,3-cyclohexanedione présente un substituant alkyl($C_1$—$C_4$)-sulfonyle ou haloalkyl($C_1$—$C_4$)-sulfonyle sur le noyau phényle.

32. Le procédé selon la revendication 31, dans lequel le substituant alkylsulfonyle ou haloalkylsulfonyle est en position 4 du noyau phényle.

33. La composition herbicide selon la revendication 28, dans lequel la 2-(2-nitrobenzoyl)-1,3-cyclohexanedione présente un substituant alkyl($C_1$—$C_4$)-sulfonyle ou haloalkyl($C_1$—$C_4$)-sulfonyle sur le noyau phényle.

34. La composition herbicide selon la revendication 33, dans lequel le substituant alkylsulfonyle ou haloalkylsulfonyle est en position 4 du noyau phényle.

35. Le procédé selon la revendication 30, dans lequel la 2-(2-nitrobenzoyl)-1,3-cyclohexanedione

présente un substituant haloalkyle en $C_1$ à $C_4$ sur le noyau phényle.

36. Le procédé selon la revendication 30, dans lequel le substituant haloalkyle est en position 4 du noyau phényle.

37. La composition herbicide selon la revendication 28, dans laquelle la 2-(2-nitrobenzoyl)-1,3-cyclohexanedione présente un substituant haloalkyle en $C_1$ à $C_4$ sur le noyau phényle.

38. La composition herbicide selon la revendication 37, dans laquelle le substituant haloalkyle est en position 4 du noyau phényle.

39. La composition herbicide selon la revendication 37, dans laquelle le radical haloalkyle est $CF_3$.

40. Le procédé selon la revendication 35, dans lequel le radical haloalkyle est $CF_3$.

41. Le procédé selon la revendication 27, dans lequel $R^1$ et $R^2$ sont des atomes d'hydrogène ou tous les deux des radicaux méthyles.

42. Le procédé selon la revendication 41, dans lequel $R^8$ est $-SO_2CH_3$, $-SO_2CH_2Cl$, $CF_3$, $-SO_2CH_3$, un atome de chlore, $-SO_2CH_2Cl$ ou $-SO_2$-n-$C_3H_7$.

43. La composition selon la revendication 28, dans lequel $R^1$ et $R^2$ sont de l'hydrogène ou tous deux un radical méthyle.

44. La composition selon la revendication 43, dans lequel $R^8$ est $-SO_2CH_3$, $-SO_2CH_2Cl$, $CF_3$, $-SO_2CH_3$, un atome de chlore, $-SO_2CH_2Cl$ ou $-SO_2$-n-$C_3H_7$.

45. Un procédé de préparation d'un composé de formule:

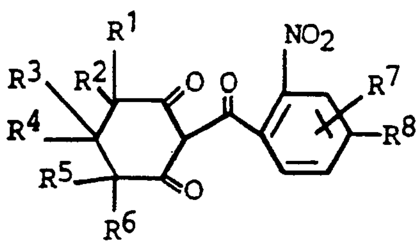

dans laquelle:

$R^1$ est un atome d'hydrogène ou un radical alkyle en $C_1$ à $C_4$;

$R^2$ est un atome d'hydrogène ou un radical alkyle en $C_1$ à $C_4$ ou un groupe $R^a$—O—C(O)— dans lequel $R^a$ est un radical alkyle en $C_1$ à $C_4$; ou

$R^1$ et $R^2$, pris ensemble, forment un groupe alkylène présentant de 3 à 6 atomes de carbone;

$R^3$ est un atome d'hydrogène ou un radical alkyle en $C_1$ à $C_4$;

$R^4$ est un atome d'hydrogène ou un radical alkyle en $C_1$ à $C_4$;

$R^5$ est un atome d'hydrogène ou un radical alkyle en $C_1$ à $C_4$;

$R^6$ est un atome d'hydrogène ou un radical alkyle en $C_1$ à $C_4$; et

$R^7$ et $R^8$, indépendamment l'un de l'autre, consistent en:

(1) un atome d'hydrogène;

(2) un atome d'halogène;

(3) un radical alkyle en $C_1$ à $C_4$;

(4) un radical alkoxy en $C_1$ à $C_4$;

(5) un groupe $-OCF_3$;

(6) un groupe cyano;

(7) un groupe nitro;

(8) un groupe haloalkyle en $C_1$ à $C_4$;

(9) un groupe $R^bSO_n$— dans lequel n est un nombre entier égal à 0, 1 ou 2 et $R^b$ est:

(a) un radical alkyle en $C_1$ à $C_4$,

(b) un radical alkyle en $C_1$ à $C_4$, substitué par un atome d'halogène ou un groupe cyano;

(c) un groupe phényle; ou

(d) un groupe benzyle;

(10) un groupe $-NR^cR^d$ dans lequel $R^c$ et $R^d$, indépendamment l'un de l'autre, consistent en un atome d'hydrogène ou un radical alkyle en $C_1$ à $C_4$;

(11) un groupe $R^eC(O)$— dans lequel $R^e$ est un radical alkyle en $C_1$ à $C_4$ ou alkoxy en $C_1$ à $C_4$; ou

(12) un groupe $SO_2NR^cR^d$ dans lequel $R^c$ et $R^d$ sont tels que définis,

à condition que $R^7$ ne soit pas lié en position 6, consistant:

(a) faire réagir une dione de formule:

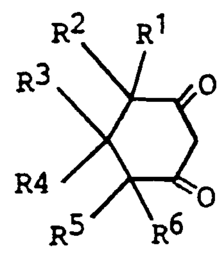

dans laquelle $R^1$ à $R^6$ sont tels que définis ci-dessus, avec un réactif benzoyle substitué de formule:

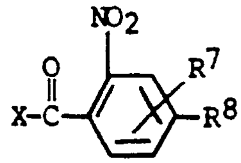

dans laquelle $R^7$ et $R^8$ sont tels que définis ci-dessus et X est un atome d'halogène ou un groupe alkyl($C_1$—$C_4$)—C(O)—O—, alcoxy($C_1$—$C_4$)—C(O)—O— ou

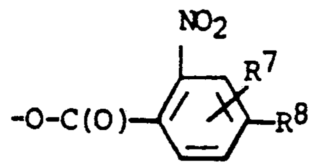

dans laquelle $R^7$ et $R^8$ de cette partie de la molécule sont identiques à ceux du réactif précité, avec au moins une mole d'une base modérée pour former un ester énolique de formule:

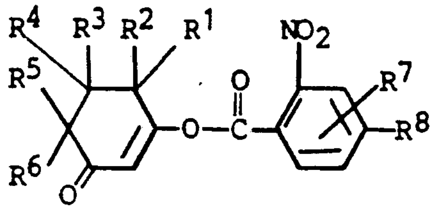

dans laquelle $R^1$ à $R^8$ sont tels que définis ci-dessus, et, b) dans l'étape (2) à faire réagir une mole de l'ester énolique intermédiaire avec une 1 à 4 moles d'une base modérée et de 0,01 mole à environ 0,5 mole ou davantage d'une source de cyanure pour former un composé de formule:

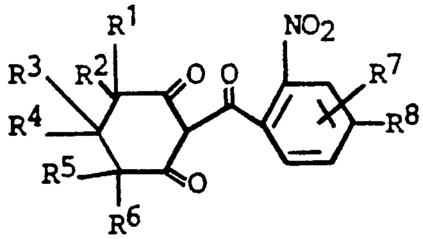

dans laquelle $R^1$ à $R^8$ sont tels que définis ci-dessus.

46. Le procédé selon la revendication 45, dans lequel X est un atome d'halogène, la base modérée consiste en tri-alkyl-($C_1$—$C_6$)amine, une pyridine, un carbonate de métal alcalin ou un phosphate de métal alcalin et la source de cyanure est un cyanure de métal alcalin, des cyanohydrines de méthyl-alkyl-($C_1$—$C_4$)-cétones, des cyanohydrines de benzaldéhyde ou d'aldéhydes aliphatiques en $C_2$ à $C_5$, des cyanohydrines, le cyanure de zinc, les cyanures de tri(alkyl-inférieur)silyle ou l'acide cyanhydrique.

47. Le procédé selon la revendication 46, dans lequel X est un atome de chlore, la base modérée consiste en tri-alkyl-($C_1$—$C_6$)amine, pyridine, carbonate de sodium ou phosphate de sodium et la source de cyanure est le cyanure de potassium, l'acétone cyanohydrine ou l'acide cyanhydrique.

**Revendications pour l'Etat contractant: AT**

1. Un procédé de préparation d'un composé de formule:

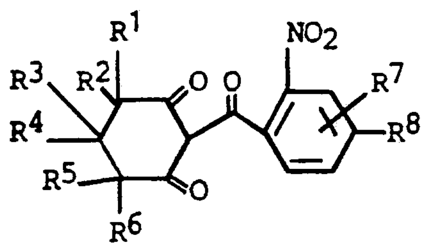

dans laquelle:

$R^1$ est un atome d'hydrogène ou un radical alkyle en $C_1$ à $C_4$;

$R^2$ est un atome d'hydrogène ou un radical alkyle en $C_1$ à $C_4$ ou un groupe $R^a$—O—C(O)— dans lequel $R^a$ est un radical alkyle en $C_1$ à $C_4$; ou

$R^1$ et $R^2$, pris ensemble, forment un groupe alkylène présentant de 3 à 6 atomes de carbone;

$R^3$ est un atome d'hydrogène ou un radical alkyle en $C_1$ à $C_4$;

$R^4$ est un atome d'hydrogène ou un radical alkyle en $C_1$ à $C_4$;

$R^5$ est un atome d'hydrogène ou un radical alkyle en $C_1$ à $C_4$;

$R^6$ est un atome d'hydrogène ou un radical alkyle en $C_1$ à $C_4$; et

$R^7$ et $R^8$, indépendamment l'un de l'autre, consistent en:

(1) un atome d'hydrogène;

(2) un atome d'halogène;

(3) un radical alkyle en $C_1$ à $C_4$;

(4) un radical alkoxy en $C_1$ à $C_4$;

(5) un groupe $—OCF_3$;

(6) un groupe cyano;

(7) un groupe nitro;

(8) un groupe haloalkyle en $C_1$ à $C_4$;

(9) un groupe $R^bSO_n$— dans lequel n est un nombre entier égal à 0, 1 ou 2 et $R^b$ est:

(a) un radical alkyle en $C_1$ à $C_4$,

(b) un radical alkyle en $C_1$ à $C_4$, substitué par un atome d'halogène ou un groupe cyano;

(c) un groupe phényle; ou

(d) un groupe benzyle;

(10) un groupe $—NR^cR^d$ dans lequel $R^c$ et $R^d$, indépendamment l'un de l'autre, consistent en un atome d'hydrogène ou un radical alkyle en $C_1$ à $C_4$;

(11) un groupe $R^eC(O)$— dans lequel $R^e$ est un radical alkyle en $C_1$ à $C_4$ ou alkoxy en $C_1$ à $C_4$; ou

(12) un groupe $SO_2NR^cR^d$ dans lequel $R^c$ et $R^d$ sont tels que définis,

à condition que $R^7$ ne soit pas lié en position 6, consistant:

(a) à faire réagir une dione de formule:

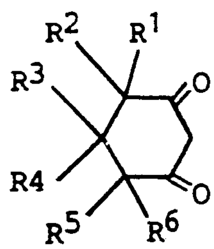

dans laquelle $R^1$ à $R^6$ sont tels que définis ci-dessus, avec un reáactif benzoyle substitué de formule:

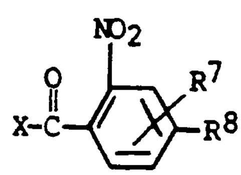

dans laquelle $R^7$ et $R^8$ sont tels que définis ci-dessus et X est un atome d'halogène ou un groupe alkyl($C_1$—$C_4$)—C(O)—O—, alcoxy($C_1$—$C_4$)—C(O)—O— ou

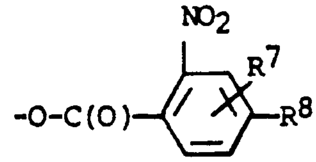

dans laquelle $R^7$ et $R^8$ de cette partie de la molécule sont identiques à ceux du réactif précité, avec au moins une mole d'une base modérée pour former un ester énolique de formule:

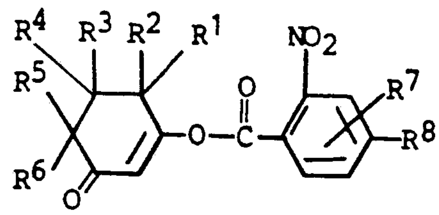

dans laquelle $R^1$ à $R^8$ sont tels que définis ci-dessus, et, b) dans l'étape (2) à faire réagir une mole de l'ester énolique intermédiaire avec une 1 à 4 moles d'une base modérée et de 0,01 mole à environ 0,5 mole ou davantage d'une source de cyanure pour former un composé de formule:

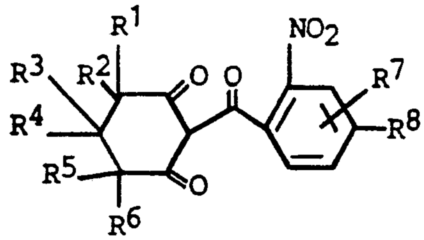

dans laquelle $R^1$ à $R^8$ sont tels que définis ci-dessus.

2. Le procédé selon la revendication 45, dans lequel X est un atome d'halogène, la base modérée consiste en tri-alkyl-($C_1$—$C_6$)amine, une pyridine, un carbonate de métal alcalin ou un phosphate de métal alcalin et la source de cyanure est un cyanure de métal alcalin, des cyanohydrines de méthyl-alkyl-($C_1$—$C_4$)-cétones, des cyanohydrines de benzaldéhyde ou d'aldéhydes aliphatiques en $C_2$ à $C_5$, des cyanohydrines, le cyanure de zinc, les cyanures de tri(alkyl-inférieur)silyle ou l'acide cyanhydrique.

3. Le procédé selon la revendication 46, dans lequel X est un atome de chlore, la base modérée consiste en tri-alkyl-($C_1$—$C_6$)amine, pyridine, carbonate de sodium ou phosphate de sodium et la source de cyanure est le cyanure de potassium, l'acétone cyanohydrine ou l'acide cyanhydrique.

4. Le procédé selon la revendication 1, caractérisé en ce que l'on prépare un composé dans lequel $R^1$ est un atome d'hydrogène ou un radical méthyle; $R^2$ est un atome d'hydrogène ou un radical méthyle; $R^3$ est un atome d'hydrogène ou un radical méthyle; $R^4$ est un atome d'hydrogène ou un radical méthyle; $R^5$ est un atome d'hydrogène ou un radical méthyle; $R^6$ est un atome d'hydrogène ou un radical méthyle; $R^7$ et $R^8$, indépendamment l'un de l'autre, consistent en (1) un atome d'hydrogène; (2) un atome d'halogène; (3) un radical alkyle en $C_1$ à $C_4$; (4) un radical alkoxy en $C_1$ à $C_4$; (5) un groupe $OCF_3$; (6) un groupe cyano; (7) un groupe nitro; (8) un radical haloalkyle en $C_1$ à $C_4$; (9) un groupe $R^bSO_n$— dans lequel n est un nombre entier égal à 0, 1 ou 2, et $R^b$ est (a) un radical alkyle en $C_1$ à $C_4$; (b) un radical alkyle en $C_1$ à $C_4$ substitué par halogène ou cyano; (c) un groupe phényle; ou (d) un groupe benzyle; (10) un groupe —$NR^cR^d$ dans lequel $R^c$ et $R^d$ indépendamment l'un de l'autre consistent en un atome d'hydrogène ou un radical alkyle en $C_1$ à $C_4$; (11) un groupe $R^eC(O)$— dans lequel $R^e$ est un radical alkyle en $C_1$ à $C_4$ ou alkoxy en $C_1$ à $C_4$; ou (12) un groupe $SO_2NR^cR^d$ dans lequel $R^c$ et $R^d$ sont tels que définis.

5. Le procédé selon la revendication 1, caractérisé en ce que l'on prépare un composé dans lequel $R^7$ et $R^8$ sont, indépendamment l'un de l'autre, un atome d'hydrogène, de chlore, de fluor, de brome, ou un groupe méthyle, méthoxy, $OCF_3$, cyano, nitro, trifluorométhyle; un groupe $R^bSO_n$— dans lequel n est un nombre entier égal à 2 et $R^b$ est un radical méthyle, chlorométhyle, trifluorométhyle, cyanométhyle, éthyle ou n-propyle; un groupe —$NR^cR^d$ dans lequel $R^c$ et $R^d$, indépendamment l'un de l'autre, consistent en un atome d'hydrogène ou un radical alkyle en $C_1$ à $C_4$; un groupe $R^eC(O)$— dans lequel $R^e$ est un radical alkyle en $C_1$ à $C_4$ ou alkoxy en $C_1$ à $C_4$, ou un groupe $SO_2NR^cR^d$ dans lequel $R^c$ et $R^d$ sont tels que définis ci-dessus et $R^7$ est en position 3.

6. Le procédé selon la revendication 1, caractérisé en ce que l'on prépare un composé dans lequel $R^7$ est un atome d'hydrogène et $R^8$ est un atome d'hydrogène, de chlore, de brome, de fluor, un groupe $CF_3$ ou $R^bSO_2$ dans lequel $R^b$ est un radical alkyle en $C_1$ à $C_4$ ou haloalkyle en $C_1$ à $C_4$.

7. Le procédé selon la revendication 1, caractérisé en ce que l'on prépare un composé dans lequel $R^1$ est un radical méthyle; $R^2$ est un radical méthyle; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un atome d'hydrogène.

8. Le procédé selon la revendication 1, caractérisé en ce que l'on prépare un compose dans lequel $R^1$ est un radical méthyle; $R^2$ est un radical méthyle; $R^3$ est un atome d'hydrogène; $R^4$ est un atome d'hydrogène; $R^5$ est un radical méthyle; $R^6$ est un atome d'hydrogène; $R^7$ est un atome d'hydrogène; et $R^8$ est un atome d'hydrogène.

9. Le procédé selon la revendication 1, caractérisé en ce que l'on prépare un composé dans lequel $R^1$ est un radical méthyle; $R^2$ est un radical méthyle; $R^3$ est un atome d'hydrogène; $R^4$ est un atome d'hydrogène; $R^5$ est un atome d'hydrogène; $R^6$ est un atome d'hydrogène $R^7$ est un atome d'hydrogène; et $R^8$ est un radical trifluorométhyle.

10. Le procédé selon la revendication 1, caractérisé en ce que l'on prépare un composé dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un radical trifluorométhyle.

11. Le procédé selon la revendication 1, caractérisé en ce que l'on prépare un composé dans lequel $R^1$ est un radical méthyle; $R^2$ est un radical méthyle; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un atome de chlore.

12. Le procédé selon la revendication 1, caractérisé en ce que l'on prépare un composé dans lequel $R^1$ est un radical méthyle; $R^2$ est un radical méthyle; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un atome de fluor.

13. Le procédé selon la revendication 1, caractérisé en ce que l'on prépare un composé dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un atome de chlore.

14. Le procédé selon la revendication 1, caractérisé en ce que l'on prépare un compose dans lequel $R^1$ est un radical méthyle; $R^2$ est un radical méthyle; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un groupe $CH_3SO_2$—.

15. Le procédé selon la revendication 1, caractérisé en ce que l'on prépare un composé dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un groupe n-$C_3H_7SO_2$.

16. Le procédé selon la revendication 1, caractérisé en ce que l'on prépare un composé dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un groupe $CH_3SO_2$.

17. Le procédé selon la revendication 1, caractérisé en ce que l'on prépare un composé dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un groupe $C_2H_5SO_2$.

18. Le procédé selon la revendication 1, caractérisé en ce que l'on prépare un composé dans lequel $R^7$ est un atome d'hydrogène.

19. Le procédé selon la revendication 5, caractérisé en ce que l'on preparé un composé dans lequel $R^7$ est un atome d'hydrogène.

20. Le procédé selon la revendication 1, caractérisé en ce que l'on prépare un composé dans lequel $R^1$ est un atome d'hydrogène; $R^2$ est un atome d'hydrogène; $R^3$ est un atome d'hydrogène; $R^4$ est un atome d'hydrogène; $R^5$ est un atome d'hydrogène; $R^6$ est un atome d'hydrogène; $R^7$ est un atome d'hydrogène; et $R^8$ est un groupe cyano.

21. Le procédé selon la revendication 1, caractérisé en ce que l'on prépare un composé dans lequel $R^1$ et $R^2$ sont des atomes d'hydrogène ou tous les deux des radicaux méthyles.

22. Le procédé selon la revendication 21, caractérisé en ce que l'on prépare un composé dans lequel $R^8$ est un groupe $—SO_2CH_3$.

23. Le procédé selon la revendication 21, caractérisé en ce que l'on prépare un composé dans lequel $R^8$ est le groupe $—SO_2CH_2Cl$.

24. Le procédé selon la revendication 1, caractérisé en ce que l'on prépare un composé dans lequel $R^8$ est le groupe $CF_3$.

25. Le procédé selon la revendication 1, caractérisé en ce que l'on prépare un composé 1, dans lequel $R^8$ est le groupe $—SO_2CH_3$.

26. Le procédé selon la revendication 1, caractérisé en ce que l'on prépare un composé dans lequel $R^8$ est un atome de chlore.

27. Le procédé selon la revendication 1, caractérisé en ce que l'on prépare un composé dans lequel $R^8$ est le groupe $—SO_2CH_2Cl$.

28. Le procédé selon la revendication 1, caractérisé en ce que l'on prépare un composé dans lequel $R^8$ est le groupe $—SO_2$-n-$C_3H_7$.

29. Procédé pour contrôler la végétation indésirable, consistant à appliquer aux zones où l'on souhaite effectuer le contrôle une quantité efficace du point de vue herbicide d'un composé selon l'une quelconque des revendications 1 à 28.

30. Une procédé de preparation d'une composition herbicide comprenant une quantité active du point de vue herbicide de 2-(2-nitrobenzoyl)-1,3-cyclohexanedione et un support ou véhicule inerte pour ce composé.

31. Le procédé selon la revendication 30, dans lequel la 2-(2-nitrobenzoyl)-1,3-cyclohexanedione est un composé selon les revendications 1 à 28.

32. Le procédé pour contrôler la végétation indésirable, consistant à appliquer aux zones où le contrôle

est souhaité une composition herbicide comprenant une 2-(2-nitrobenzoyl)-1,3-cyclohexanedione active du point de vue herbicide et un support ou véhicule inerte pour ce composé.

33. Le procédé selon la revendication 32, dans lequel la 2-(2-nitrobenzoyl)-1,3-cyclohexanedione présente un substituant alkyl($C_1$—$C_4$)-sulfonyle ou haloalkyl($C_1$—$C_4$)-sulfonyle sur le noyau phényle.

34. Le procédé selon la revendication 33, dans lequel le substituant alkylsulfonyle ou haloalkylsulfonyle est en position 4 du noyau phényle.

35. La composition herbicide selon la revendication 30, dans lequel la 2-(2-nitrobenozyl)-1,3-cyclohexanedione présente un substituant alkyl($C_1$—$C_4$)-sulfonyle ou haloalkyl($C_1$—$C_4$)sulfonyle sur le noyau phényle.

36. La composition herbicide selon la revendication 35, dans lequel le substituant alkylsulfonyle ou haloalkylsulfonyle est en position 4 du noyau phényle.

37. Le procédé selon la revendication 32, dans lequel la 2-(2-nitrobenzoyl)-1,3-cyclohexanedione présente un substituant haloalkyle en $C_1$ à $C_4$ sur le noyau phényle.

38. Le procédé selon la revendication 32, dans lequel le substituant haloalkyle est en position 4 du noyau phényle.

39. La composition herbicide selon la revendication 30, dans laquelle la 2-(2-nitrobenzoyl)-1,3-cyclohexanedione présente un substituant haloalkyle en $C_1$ à $C_4$ sur le noyau phényle.

40. La composition herbicide selon la revendication 39, dans laquelle le substituant haloalkyle est en position 4 du noyau phényle.

41. La composition herbicide selon la revendication 39, dans laquelle le radical haloalkyle est $CF_3$.

42. Le procédé selon la revendication 37, dans lequel le radical haloalkyle est $CF_3$.

43. Le procédé selon la revendication 29, dans lequel $R^1$ et $R^2$ sont des atomes d'hydrogène ou tous les deux des radicaux méthyles.

44. Le procédé selon la revendication 43, dans lequel $R^8$ est $—SO_2CH_3$, $—SO_2CH_2Cl$, $CF_3$, $—SO_2CH_3$, un atome de chlore, $—SO_2CH_2Cl$ ou $—SO_3$-n-$C_3H_7$.

45. La composition selon la revendication 30, dans lequel $R^1$ et $R^2$ sont de l'hydrogène ou tous deux un radical méthyle.

46. La composition selon la revendication 45, dans lequel $R^8$ est $—SO_2CH_3$, $—SO_2CH_2Cl$, $CF_3$, $—SO_2CH_3$, un atome de chlore, $—SO_2CH_2Cl$ ou $—SO_3$-n-$C_3H_7$.